# EUROPEAN PATENT APPLICATION

(11) **EP 1 640 010 A1**
(43) Date of publication of application: **29.03.2006**
(21) Application number: 04747073.7
(22) Date of filing: 30.06.2004
(51) Int. Cl.: A61K 31/663, A61K 31/5025, A61K 31/662, A61K 45/00, A61P 1/02, A61P 19/00, A61P 19/02, A61P 19/04, A61P 19/08, A61P 19/10, A61P 29/00, A61P 35/00, A61P 35/04

(54) **AGENT INDUCING INCREASE IN BONE MASS**

(30) Priority: 01.07.2003 JP 2003189837; 18.12.2003 JP 2003420912
(71) Applicant: Astellas Pharma Inc., Tokyo 103-8411 (JP)
(72) Inventor: KANOH, Hiroyuki Astellas Pharma Inc., Chuo-ku, Tokyo 103-8411 (JP); TAKAHASHI, Koichiro Astellas Pharma Inc., Chuo-ku, Tokyo 103-8411 (JP); NARA, Hiromi Astellas Pharma Inc., Chuo-ku, Tokyo 103-8411 (JP); YAMANOI, Tatsuhiko Astellas Pharma Inc., Chuo-ku, Tokyo 103-8411 (JP); FUKUSHIMA, Shinji Astellas Pharma Inc., Chuo-ku, Tokyo 103-8411 (JP); NAITO, Ryo Astellas Pharma Inc., Chuo-ku, Tokyo 103-8411 (JP); IGARASHI, Susumu Astellas Pharma Inc., Chuo-ku, Tokyo 103-8411 (JP)
(74) Representative: HOFFMANN EITLE
(86) International application number: PCT/JP2004/009604
(87) International publication number: WO 2005/002590

(57) **Abstract**

Osteoporosis and the like metabolic bone diseases have a high frequency of causing lumbago and the like pains and bone fracture, due to lowering of the bone strength caused by the reduction of bone mass. Accordingly, great concern has been directed toward the creation of a drug which can increase the bone mass and bone strength by controlling the whole bone metabolism.

The pharmaceutical composition or combination product of the invention comprising a non-living body-derived non-peptide osteoblast differentiation promoting compound and a bisphosphonate having a bone resorption inhibitory action is useful as a bone mass increasing inducer which can increase the bone mass and/or bone strength by controlling the bone metabolism.

## Description

### TECHNICAL FIELD

The present invention relates to a pharmaceutical composition or combination useful for preventing and treating a metabolic bone disease and the like bone diseases, comprising a combination of a non-living body-derived non-peptide osteoblast differentiation promoting compound, particularly a nitrogen-containing heterocyclic compound represented by a general formula (I) which is described later or a salt thereof, with a bisphosphonate.

### BACKGROUND ART

A normal bone metabolism involves an equilibrium between the level of bone resorption by osteoclasts and the level of bone formation by osteoblasts, by which a homeostasis is maintained. A metabolic bone disease is considered to be developed once such a balance between the bone resorption and the bone formation is lost. This disease includes osteoporosis, osteitis fibrosa (hyperparathyroidism), osteomalacia and further Paget's disease which affects the parameters of systemic bone metabolism. As the osteoporosis which causes low back pain and the like pains and bone fracture and the like, there are known primary osteoporosis and secondary osteoporosis which is induced by a steroid administration and the like. Primary osteoporosis can be classed as either high bone turnover type (type I) which is observed frequently in postmenopausal women, or senile low bone turnover type (type II). Particularly, it has been reported that deformation of vertebral body and bone fracture of femoral neck are frequent in the case of the senile osteoporosis, because of the reduction of the bone mass of not only cancellous bone but also cortical bone due to reduction of the bone-forming ability (Riggs BL *et al., New Eng. J. Med*., 314, 1676 (1986)). Bone fracture in the aged is serious because it leads to the whole body weakness and dementia.

It is known that various living body-derived physiologically active substances are concerned in this bone remodeling consisting of bone resorption and bone formation; for example, parathyroid hormone (PTH), PTHrP (PTH-related protein), TNFα, M-CSF, active type vitamin D3 (1α, 25 (OH)₂D₃), prostaglandin, RANKL (receptor activator of NF-κB ligand) and the like are known as factors which are concerned in the bone resorption acceleration or osteoclast differentiation acceleration, and calcitonin, estrogen, OPG (osteoprotegerin) and the like as factors concerned in the bone resorption inhibition, PTH, BMP (bone morphogenetic protein), TGF **β** (transforming growth factor β), prostaglandin and the like as factors concerned in the bone formation, and active type vitamin D3 (1α, 25(OH)₂D₃), PTH and the like as factors concerned in the calcium metabolism of the whole body. Particularly, estrogen, calcitonin, active type vitamin D3 and the like are used in the clinical field as therapeutic and preventive agents for metabolic bone diseases including osteoporosis. However, since these agents have a mechanism to increase bone density by mainly controlling bone resorption or calcium metabolism, it is said that the effect is not sufficient for senile osteoporosis and the like in which the bone formation ability is reduced. Recently, favorable bone density improving action of a PTH having bone formation promoting action has been reported (*J*. *Clin. Endocrinol. Metab.,* 82, 620- 628 (1997)), and this substance is expected as a new anti-osteoporosis agent.

On the other hand, bisphosphonate is a compound having a structure similar to that of pyrophosphoric acid which is a calcium deposition inhibitor, and since it shows a bone resorption inhibitory action by inhibiting the function of osteoclast, this is used as a drug for treating osteoporosis and the like metabolic bone diseases. For example, it has been reported that an alendronate or a risedronate as a typical bisphosphonate reduced generation frequency of bone fracture of femoral neck in a large scale clinical test carried out using postmenopausal female osteoporosis patients (Non-patent Reference 1: *New England Journal of Medicine,* 333(22), pp. 1437 - 1443 (1995) and Non-patent Reference 2: *New England Journal of Medicine,* 344(5), pp. 333 - 340 (2001)). However, since a result was disclosed stating that its effect was not significant in aged patients of 80 years old or more (Non-patent Reference 2), it was suggested that sufficient effect cannot be expected in senile osteoporosis.

Up to now, some attempts have been made to combined use a living body-derived physiologically active substance having bone formation promoting action and a bisphosphonate having bone resorption inhibitory action. For example, a method for treating bone metabolism disorders by administering a PTH for a prolonged period of time after long-term administration of a bisphosphonate (Patent Reference 1: International Publication 96/07417). However, there is a report stating that the effect by simultaneous combined administration of both agents is not always clear (Non-patent Reference 3: R. Neer *et al*., Bone, 32(5), S 69 (2003)). In addition, it is known that physiologically active substance prostaglandin E2 (PGE2), which is clinically put into practical use as a vasodilator and a labor inducer, also has the bone forming action and bone resorption action as one of its many activities, and it has been reported that the bone mass was increased when PGE2 was administered in combination with an alendronate as the bisphosphonate (Non-patent Reference 4: *Journal of Bone and Mineral Research,* 8(7), (1993), p. 871-9.

However, because of the reason that already known living body-derived physiologically active substances do not have sufficient effects, being peptides, their administration methods are limited, or they accompany undesirable actions because of their various activities in the living body, concern has been directed toward the creation of a new type bone mass increasing inducer which is not a living body-derived physiologically active substance, but from which a non-peptide bone fracture inhibitory activity can be expected also in the case of senile osteoporosis having reduced bone forming ability.

Recently, non-peptide compounds which show an alkaline phosphatase (ALP) inducing activity and have osteoblast differentiation promoting action have been reported. For example, there are reports on benzothiepin derivatives (Patent References 2 to 4: JP-A-8-231569, JP-A-2000-109480 and JP-A-9-263545 = WO 9639134), chromone derivatives (Patent Reference 5: JP-A-2001-139571 = WO 0116127), thiophene derivatives (Patent References 6 to 11: JP-A-2002-47184, JP-A-2002-255971 = WO 01174823, JP-A-2000-309591, JP-A-10-130271 = WO 9809958, JP-A-2001-151775 and JP-A-2001-151774), purine derivatives (Non-patent Reference 5: *J. Am. Chem. Soc.,* 2002, 124, 14520 - 14521), *N-*quinolylanthranilic acid derivatives (Patent Reference 12: JP-A-9-188622) and the like. Though it is disclosed that these compounds having osteoblast differentiation promoting action show bone formation promoting action and are useful in treating metabolic bone diseases and bone fracture, the clinical usefulness is yet unknown. In addition, there is a description in some of the patent gazettes disclosing these compounds, stating that these osteoblast differentiation promoting compounds can be used concomitantly with other bone mass increasing inducers or bone resorption inhibitors, and bisphosphonate is included in the examples of other concomitant drugs (e.g., the aforementioned Patent References 2, 3, 5 and 7). However, there is no disclosure on an illustrative example in which an osteoblast differentiation promoting compound was used concomitantly with a bisphosphonate, or on the effect of the concomitant use.

In this connection, there are the following reports with regard to triazolopyridazine derivatives as the compounds having similar structures to that of the nitrogen-containing heterocyclic compound represented by a general formula (I) of the invention which is described below (symbols in the description represent the symbols in the general formula (I) described below). However, any of these references and patent specifications does not contain any disclosure or suggestion of an osteoblast differentiation promoting action or an osteogenesis-promoting action.
(1) An antibacterial compound wherein Ra and Rb are taken together with an adjacent N atom to form a piperidino, E is a single bond, and R is piperidino (Patent Reference 13: US Patent 3,957,766); a method for synthesizing a compound wherein R is an unsubstituted phenyl (Non-patent Reference 6: *Tetrahedron*, 22(7), 2073-9 (1966)); a structure of a compound wherein R is p-(trifluoromethyl)phenyl or *p*-chlorophenyl (Non-patent Reference 7: CAS Registry File, RN = 289651-67-8 or 202820-26-6); and a compound wherein R is *o*-nitrophenyl (Non-patent Reference 8: a catalog of SPECS, Holland, (Refcode: AG-690/3073051) are respectively disclosed.
(2) A triazolopyridazine derivative having a bronchodilating effect which is a compound wherein Ra and Rb are taken together with an adjacent N atom to form a 4-methyl-1-piperazinyl, E is a single bond, and R is an unsubstituted phenyl, *p*-methylphenyl, *m*-methylphenyl, *p-*methoxyphenyl, *m*-chlorophenyl, *p*-chlorophenyl or *m-*nitrophenyl (Patent Reference 14: German Patent 2,444,322 and Patent Reference 15: JP-A-50-58092) is disclosed.
(3) An antibacterial compound wherein R is an imidazolyl which may have a substituent group (Patent References 16, 17 and 18: German Patent publications 2,261,693, 2,254,873 and 2,215,999); and an antibacterial compound wherein R is 5-nitro-2-furyl or 5-nitro-2-thienyl (Patent References 19, 20 and 21 German Patent publications 2,161,586, 2,161,587 and 2,113,438) are disclosed.
(4) The structure of a compound wherein Ra is H, Rb is cyclopropyl, E is a single bond and R is a *p-*(trifluoromethyl)phenyl (Non-patent Reference 9: CAS Registry File, RN = 289651-68-9) is disclosed.
(5) An anti-hypertensive compound wherein Ra is a methyl, Rb is a 2-hydroxy-propyl, E is a single bond and R is a 3-pyridyl (Non-patent Reference 10: *Farmaco. Ed. Sci.,* 34(4), 299-310 (1979)) is disclosed.

In the case of a metabolic bone disease such as osteoporosis, the bone strength is reduced due to reduction of the bone mass so that the frequency of causing low back pain and the like pains and bone fracture is high, and particularly in the aged patients having reduced bone forming ability, the prognosis is markedly poor when bone fracture of femoral neck is caused. Thus, strong concern has been directed toward the creation of an agent which can increase the bone mass and/or bone strength by controlling the whole bone metabolism. Particularly, concern has been directed toward the creation of a new type bone mass increasing inducer, which is a non-living body-derived and non-peptide substance and from which an excellent bone fracture inhibitory activity can be expected even in the case of the senile osteoporosis of the low bone turnover type (type II).

### DISCLOSURE OF THE INVENTION

As a result of carrying out intensive studies for the purpose of developing a therapeutic agent having a bone formation-stimulating effect by promoting the functions of osteoblasts, the present inventors discovered that a novel nitrogen-containing heterocyclic compound which is shown later exhibits an excellent osteoblast differentiation promoting activity and thus can serve as a preventive or therapeutic agent for a metabolic bone disease, as a bone formation promoter similar to the case of the already known non-living body-derived non-peptide osteoblast differentiation promoting compounds, and its patent application was carried out recently (International Publication 03/074525.

In addition, as a result of carrying out intensive studies on an agent which can increase the bone mass and/or bone strength by controlling the bone metabolism, it was found that, when the aforementioned nitrogen-containing heterocyclic compound (I) as an excellent osteoblast differentiation promoting compound is used concomitantly with a bisphosphonate having born resorption action, bone mass and/or bone strength of the femoral neck is reinforced synergistically or more than additively, and for example, in the test using an osteoporosis model (rat OVX) which is described later, excellent bone mass increase inducing effect surpassing the sham operation group can be attained, thereby accomplishing the invention.

That is, the present invention are:
(1) a pharmaceutical composition which is a bone mass increasing inducer comprising a) a non-living body-derived non-peptide osteoblast differentiation promoting compound as the first component and b) a bisphosphonate as the second component,
(2) a pharmaceutical composition which is a bone mass increasing inducer comprising a) a nitrogen-containing heterocyclic compound represented by the following general formula (I), which is a novel non-living body-derived non-peptide osteoblast differentiation promoting compound, or a salt thereof as the first component and b) a bisphosphonate as the second component,
(3) the aforementioned pharmaceutical composition, wherein the bone mass increasing inducer is a preventive or therapeutic agent for a metabolic bone disease,
(4) the aforementioned pharmaceutical composition, wherein the bone mass increasing inducer is a preventive or therapeutic agent for a low bone turnover type (type II) osteoporosis,
(5) a combination product which is a bone mass increasing inducer consisting of two pharmaceutical preparations of a pharmaceutical preparation containing a non-living body-derived non-peptide osteoblast differentiation promoting compound as the first pharmaceutical preparation and a bisphosphonate as the second pharmaceutical preparation, wherein said first and second pharmaceutical preparations are administered simultaneously or separately,
(6) the aforementioned combination product, wherein the first pharmaceutical preparation is a pharmaceutical preparation comprising a nitrogen-containing heterocyclic compound represented by the following general formula (I) or a salt thereof,
(7) the aforementioned combination product which is a kit comprising at least two pharmaceutical preparations of a pharmaceutical preparation containing a non-living body-derived non-peptide osteoblast differentiation promoting compound as the first pharmaceutical preparation and a bisphosphonate as the second pharmaceutical preparation,
(8) an agent for reinforcing the bone mass increasing effect of a non-living body-derived non-peptide osteoblast differentiation promoting compound, which comprises a bisphosphonate as the active ingredient,
(9) an agent for reinforcing the bone mass increasing effect of a bisphosphonate, which comprises a non-living body-derived non-peptide osteoblast differentiation promoting compound as the active ingredient,
(10) use of a non-living body-derived non-peptide osteoblast differentiation promoting compound and a bisphosphonate for producing a drug which is a bone mass increasing inducer,
(11) use of a non-living body-derived non-peptide osteoblast differentiation promoting compound for producing a drug which reinforces bone mass increasing effect of a bisphosphonate,
(12) use of a bisphosphonate for producing a drug which reinforces bone mass increasing effect of a non-living body-derived non-peptide osteoblast differentiation promoting compound,
(13) a method for preventing or treating a metabolic bone disease which accompanies reduction of the bone mass and/or bone strength of the patient, which comprises administering an effective amount of a non-living body-derived non-peptide osteoblast differentiation promoting compound and an effective amount of a bisphosphonate, simultaneously or separately,
(14) the aforementioned method, wherein the metabolic bone disease which accompanies reduction of the bone mass and/or bone strength of the patient is a low bone turnover reducing type (type II) osteoporosis, and
(15) a method for inducing bone mass gain of a patient, which comprises administering an effective amount of a non-living body-derived non-peptide osteoblast differentiation promoting compound and an effective amount of a bisphosphonate, simultaneously or separately, to a patient who requires increase of the bone mass and/or bone strength. 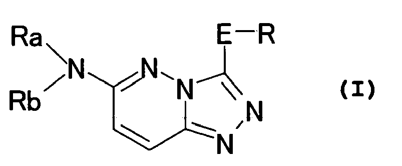
(Symbols in the formula have the following meanings,
Ra and Rb: the same or different and each represent H; CO-lower alkyl; SO₂-lower alkyl; an optionally substituted cycloalkyl; an optionally substituted aryl; or a lower alkyl which may have 1 to 3 substituents selected from the group consisting of an optionally substituted cycloalkyl, an optionally substituted aryl, an optionally substituted 4- to 8-membered monocyclic saturated or partially unsaturated heterocyclic ring, CO-lower alkyl, SO₂-lower alkyl, OR¹, SR¹, NR¹R², a halogen, NO₂, CN and COOR¹; provided that at least one of Ra and Rb represent a group other than H; or,
Ra and Rb taken together with an adjacent N atom form a 4- to 8-membered saturated or partially unsaturated heterocyclic ring containing 1 or 2 N atoms as heteroatoms, said heterocyclic ring may be fused with a benzene ring or a cycloalkyl ring and may have a bridge and may form a spiro ring, and said heterocyclic ring may have from 1 to 5 substituent groups,
E: a single bond, a C₁₋₃ alkylene, vinylene (-C=C-) ethynylene (-C≡C-) , CO, NR³, CH₂-J, CONR⁴ or NR⁵CO,
J: O, S, NR⁶, CO, SO or SO₂,
R: an optionally substituted aryl, an optionally substituted heteroaryl, an optionally substituted cycloalkyl, an optionally substituted cycloalkenyl or an optionally substituted 4- to 8-membered monocyclic saturated or partially saturated heterocyclic ring,
R¹ to R⁶: the same or different and each denotes H or a lower alkyl;
with the proviso that the following compounds are excluded:
(1) a compound wherein Ra and Rb taken together with an adjacent N atom form a piperidino, E is a single bond and R is a piperidino, unsubstituted phenyl, *p-*(trifluoromethyl)phenyl, *p*-chlorophenyl or *o*-nitrophenyl,
(2) a compound wherein Ra and Rb taken together with an adjacent N atom form a 4-methyl-1-piperazinyl, E is a single bond, and R is an unsubstituted phenyl, *p-*methylphenyl, *m*-methylphenyl, *p*-methoxyphenyl, *m-*chlorophenyl, *p*-chlorophenyl or *m*-nitrophenyl,
(3) a compound wherein R is an optionally substituted imidazolyl, 5-nitro-2-furyl or 5-nitro-2-thienyl,
(4) a compound wherein Ra is H, Rb is cyclopropyl, E is a single bond and R is a *p*-(trifluoromethyl)phenyl, and
(5) a compound wherein Ra is a methyl, Rb is a 2-hydroxypropyl, E is a single bond and R is a 3-pyridyl).

### BRIEF DESCRIPTION OF THE DRAWINGS

Fig. 1 is a graph showing a result of the femoral bone strength (three-point bending test) in Test 1 of Example 2. In this connection, the ***, ** and * show that results of Student's t-test based on the control group 1 are p<0.005, p<0.01 and p<0.05, respectively.
Fig. 2 is a graph showing a result of the femoral cortical bone mineral density (pQCT) in Test 1 of Example 2. In this connection, the ***, ** and * show that results of Student's t-test based on the control group 1 are p<0.005, p<0.01 and p<0.05, respectively.
Fig. 3 is a graph showing a result of the femoral cortical bone thickness (pQCT) in Test 1 of Example 2. In this connection, the ***, ** and * show that results of Student's t-test based on the control group 1 are p<0.005, p<0.01 and p<0.05, respectively.
Fig. 4 is a graph showing urinary deoxypyridinoline value (urine volume correction) in Test 1 of Example 2.
Fig. 5 is a graph showing a result of the tibial cortical bone thickness (pQCT) in Test 2 of Example 2. In this connection, the * shows that result of Student's t-test based on the control group 1 is p<0.05.
Fig. 6 is a graph showing a result of the tibial cortical bone mineral density (pQCT) in Test 2 of Example 2.
Fig. 7 is a graph showing urinary deoxypyridinoline value (urine volume correction) in Test 2 of Example 2. In this connection, the ***, ** and * show that results of Student's t-test based on the control group 1 are p<0.005, p<0.01 and p<0.05, respectively.

### BEST MODE FOR CARRYING OUT THE INVENTION

The following describes the invention in detail.

According to the invention, the "non-living body-derived non-peptide osteoblast differentiation promoting compound" means a low molecular organic compound which promotes functions of osteoblast by promoting differentiation induction into osteoblast or of the osteoblast itself, and distinctively shows the ALP activity promoting action of osteoblast, as its index, wherein it does not include living body-derived already existing physiologically active substances or analogues thereof and it also does not include peptide compounds. In this connection, since it has been reported that the ALP increases from the initial stage of the function expression of osteoblast (*Journal of Cellular Physiology,* vol. 143, 420 (1990)) and is concerned in the bone formation by osteoblast *(Saibo Kogaku* (Cell Engineering), vol. 13, no. 12, 1062 (1994) and *J. Clin. Invest.*, 89, 1974 (1992)), the ALP is valued as the index of bone formation promotion by the promotion of osteoblast differentiation induction.

Illustratively, the "osteoblast differentiation promoting compound" is a compound in which its possession of ALP promoting activity was confirmed by an ALP activity measuring test which uses osteoblasts by employing a measuring method similar to the method of Lowry et al. (*Journal of Biological Chemistry*, vol. 207, p. 19 (1954)). Those which show a promoting effect of 150% or more compared to a control at a concentration of 10 µM or less, in an ALP activity measuring test on at least one osteoblast or a cell having the ability to differentiate into osteoblast, are desirable, and those which show a promoting effect of 200% or more are more desirable. Particularly desirable is a compound which shows a promoting effect of 300% or more at a concentration of 300 nM. In this connection, examples of the cell which can be used in the ALP activity measuring test include a mouse calvaria-derived osteoblastic cell line MC3T3-E1, rat neonatal calvaria-derived cell lines ROB-C26, C8a, C11, C20 and C23, a rat osteosarcoma cell line ROS17/2.8, a human osteoblast NHOst, a mouse undifferentiated mesenchymal cell line C3H10T1/2, a human mesenchymal stem cell hMSC, and other various species (mouse, rat, human and the like)-derived osteoblasts, bone cells, undifferentiated mesenchymal stem cells, bone marrow cells and the like, and the aforementioned ALP promoting activity is confirmed in these cells.

Several non-living body-derived non-peptide osteoblast differentiation promoting compounds have so far been reported, of which, for example, benzothiepin derivatives (Patent References 2 to 4), chromone derivatives (Patent Reference 5), thiophene derivatives (Patent References 6 to 11), purine derivatives (Non-patent Reference 5), *N*-quinolylanthranilic acid derivatives (Patent Reference 12) and the like are suitable as the non-peptide osteoblast differentiation promoting compounds of the invention. These compounds can be easily obtained by the methods described in said Patent References or Non-patent References. In this connection, the ALP promoting activities of these compounds are as described in said Patent References or Non-patent References.

Also, a compound showing the ALP activity and capable of being used as the non-peptide osteoblast differentiation promoting compounds of the invention can be found by subjecting novel synthetic compounds, various compounds which are on the market or registered in chemical file, or a group of compounds obtained by combinatorial chemistry techniques, to the ALP activity measuring test described in Example 1 which is described later. In addition, a non-peptide osteoblast differentiation promoting compound having more superior activity can also be obtained by chemically modifying the thus found compound.

In addition, the aforementioned nitrogen-containing heterocyclic compound shown by the general formula (I) found by the present inventors (to be referred to as compound (I) hereinafter) or a salt thereof is possessed of good osteoblast differentiation promoting action and therefore is particularly desirable as the non-peptide osteoblast differentiation promoting compound of the invention.

The compound (I) is described below in detail.

As used herein, a "lower" means, unless otherwise specified, a straight or branched carbon chain having from 1 to 6 carbon atoms. As the "lower alkyl", methyl, ethyl and propyl are particularly desirable. In this specification, "Alk" is an abbreviation of "lower alkyl".

An "aryl" is preferably a C₆-₁₄ monocyclic to tricyclic aryl group. More preferably, it is a phenyl or naphthyl group, particularly, a phenyl group. It is also possible that a phenyl group is fused with a C₅₋₈ cycloalkyl group to form, for example, an indanyl or tetrahydronaphthyl group.

A "cycloalkyl" is preferably a C₃₋₁₄ cycloalkyl group, which may have bridge(s). More preferably, it is a C₃₋₁₀ cycloalkyl group, particularly, a cyclopentyl, cyclohexyl and cycloheptyl group. A "cycloalkenyl" is a group having 1 or 2 double bonds in the aforementioned "cycloalkyl" ring.

A "4- to 8-membered monocyclic saturated or partially unsaturated heterocyclic ring" is a 4- to 8-membered monocyclic saturated heterocyclic ring having from 1 to 4 heteroatoms selected from N, S and O, which may have bridge(s) and may partially have an unsaturated bond. Preferably, it is tetrahydropyranyl, tetrahydrofuranyl, pyrrolidinyl, piperidinyl, 1,2,3,6-tetrahydropyridyl, homopiperidinyl, piperazinyl, homopiperazinyl, quinucridinyl and morpholinyl group.

A "heteroaryl" is a 5- or 6-membered monocyclic heteroaryl group having from 1 to 4 heteroatoms selected from N, S and O, which may be fused with a benzene ring or a 5- or 6-membered monocyclic heteroaryl to form a bi- or tri-cyclic heteroaryl group, which may be saturated partially. Such a 5- or 6-membered heteroaryl is preferably a furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidinyl, pyridazinyl, pyrazinyl and triazinyl group, while a bi- or tri-cyclic heteroaryl is preferably a benzofuranyl, benzothienyl, benzothiadiazolyl, benzothiazolyl, benzoxazolyl, benzoxadiazolyl, benzoimidazolyl, indolyl, isoindolyl, indazolyl, quinolyl, isoquinolyl, cinnolinyl, quinazolinyl, quinoxalinyl, benzodioxolyl, pyrazinopyridyl, triazolopyridyl, naphthylidinyl and imidazopyridyl group. As a partially saturated heteroaryl, 1,2,3,4-tetrahydroquinolyl group and the like can be exemplified. More preferred as the heteroaryl is a pyridyl, pyrimidinyl, furyl, thienyl, thiazolyl, quinolyl, benzofuranyl, benzothienyl, indolyl, imidazopyridyl and naphthylidinyl group, especially a pyridyl group.

A substituent(s) on an "optionally substituted aryl", and "optionally substituted heteroaryl", "optionally substituted cycloalkyl", "optionally substituted cycloalkenyl", "optionally substituted 4- to 8-membered monocyclic saturated or partially unsaturated heterocyclic ring" is preferably the same or different 1 to 5 substituent groups selected from Group B shown below, more preferably groups selected from Group B1, especially a halogen, O-Alk and S-Alk.

Group B: An Alk which may have 1 to 4 substituent groups selected from Group G, halogen, NR¹R², NR¹CO-Alk, NO₂, CN, OR¹, -O-(Alk having 1 to 4 substituent groups selected from Group G), SR¹, -S-halogeno Alk, -O-CO-Alk, COOR¹, COR¹, CONR¹R², SO-Alk, SO₂-Alk, SO₂NR¹R², P(=O)(OR¹)₂, -O-CH₂-O-, -O-(CH₂)₂-O-, aryl which may have 1 to 4 substituent groups selected from Group D, heteroaryl which may have 1 to 4 substituent groups selected from Group D, -O-(aryl which may have 1 to 4 substituent groups selected from Group D), 4- to 8-membered monocyclic saturated or partially unsaturated heterocyclic ring which may have 1 to 4 substituent groups selected from Group D, cycloalkyl and -O-cycloalkyl. In these groups, R¹ and R² are as defined above; "Group D" consists of Alk, halogen, halogeno Alk, NR¹R², NO₂, CN, OR¹ and SR¹; "Group G" consists of halogen, NR¹R², CN, COOR¹, OR¹, SR¹, 4- to 8-membered monocyclic saturated or partially unsaturated heterocyclic ring which may have 1 to 4 substituent groups selected from Group D, aryl which may have 1 to 4 substituent groups selected from Group D and heteroaryl which may have 1 to 4 substituent groups selected from Group D; a "halogen" is I, Br, F and Cl; and a "halogeno Alk" is a lower alkyl substituted by 1 or more halogen atoms (especially CF₃), respectively. The same applies analogously to the followings.

Group B1: Alk, halogen, halogeno Alk, NR¹R², NO₂, CN, OR¹ , -O-halogeno Alk, SR¹, COOR¹, CONR¹R² , SO₂Alk, 4- to 8-membered monocyclic saturated or partially unsaturated heterocyclic ring, phenyl and phenoxy group.

A "4- to 8-membered saturated or partially unsaturated heterocyclic ring having 1 or 2 N atoms as heteroatoms" which may be formed from Ra and Rb taken together with an adjacent N atom may for example be a 4- to 8-membered monocyclic saturated or partially unsaturated heterocyclic ring having 1 or 2 N atoms as ring atoms with the rest of the ring atoms being C atoms. Such a heterocyclic ring may form a fused ring together with a benzene ring or a C₅₋₈ cycloalkyl ring, may have bridge(s), and may form a spiro ring. Preferably, it is pyrrolydinyl, piperidinyl, homopiperidinyl, piperazinyl, pyrazolidinyl, imidazolidinyl, homopiperazinyl, perhydroazocinyl, pyrrolinyl, imidazolinyl, pyrazolinyl, 1,2,3,6-tetrahydropyridyl, 1,2-dihydropyridyl, tetrahydropyridazinyl, tetrahydropyrazinyl, 1,4,5,6-tetrahydropyrimidinyl, indolinyl, isoindolinyl, 1,2,3,4-tetrahydroquinolyl, 1,2,3,4-tetrahydroisoquinolyl, 3-azabicyclo[3.2.1]octyl, 8-azabicyclo[3.2.1]octyl, 3-azabicyclo[3.2.2]nonyl, 3-azabicyclo[3.3.1]nonyl, 7-azabicyclo[2.2.1]heptyl, isoquinucridinyl, 3-azabicyclo[3.3.2]decanyl, 3-azaspiro[5.5]undecanyl, 2-azaspiro[4.5]decanyl, 2-azaspiro[4.4]nonyl, 8-azaspiro[4.5]decanyl and the like groups. It is more preferably a 4- to 8-membered monocyclic saturated or partially unsaturated heterocyclic ring which may have bridge(s), especially, pyrrolidinyl, piperidinyl, homopiperidinyl, perhydroazocinyl, 1,2,3,6-tetrahydropyridyl, 3-azabicyclo[3.2.1]octyl, 8-azabicyclo[3.2.1]octyl, 3-azabicyclo[3.2.2]nonyl and 3-azabicyclo[3.3.1]nonyl groups. A piperidyl group is especially preferred.

Such a heterocyclic ring may have a substituent(s) , and such a substituent(s) is preferably 1 to 5 substituent groups selected from Group B listed above. More preferably, it is 1 to 5 substituent groups selected from (Alk which may have substituent group(s) selected from COOR¹, OR¹ and phenyl), halogen, NR¹R², CN, OR¹, -O- (Alk which may have substituent group(s) selected from COOR¹, OR¹ and phenyl), SR¹ , COOR¹, CONR¹R² and phenyl, especially 1 or 2 substituent groups selected from Alk, halogen, OR¹ and COOR¹.

Among the compounds (I), those preferred are listed below.
(1) A compound wherein -NRaRb forms a 4- to 8-membered saturated or partially unsaturated heterocyclic ring having 1 or 2 N atoms as heteroatoms which may be fused with a benzene ring or a cycloalkyl ring, which may have bridge(s), or which may form spiro ring(s) and which may have 1 to 5 substituent groups selected from Group B; E is a single bond, C₁₋₃ alkylene, vinylene, ethynylene, CONH, CH₂NH, CH₂O or CH₂S; and R is aryl which may have 1 to 5 substituent groups selected from Group B or heteroaryl which may have 1 to 5 substituent groups selected from Group B.
(2) A compound wherein E is a single bond, C₁₋₃ alkylene, vinylene or ethynylene; and R is an aryl having 1 to 5 substituent groups selected from Group B1 or heteroaryl having 1 to 5 substituent groups selected from Group B1.
(3) A compound wherein -NRaRb is a 4- to 8-membered monocyclic saturated or partially unsaturated heterocyclic ring which may have one N atom as a ring heteroatom and may have a bridge, and may have 1 or 2 substituent groups selected from Alk, halogen, OR¹ and COOR¹; E is a single bond; and R is a phenyl having, at its m-position, a substituent group selected from a halogen, O-Alk and S-Alk or a pyridyl having, at its 6-position, a substituent group selected from a halogen, O-Alk and S-Alk.

An especially preferred compound is nitrogen-containing heterocyclic compounds listed below or their salts.

6-Azocan-1-yl-3-(6-methoxypyridin-2-yl)-1,2,4-triazolo[4,3-b]pyridazine, 6-azepan-1-yl-3-(6-bromopyridin-2-yl)-1,2,4-triazolo[4,3-b]pyridazine, 3-(3-methoxyphenyl)-6-(piperidin-1-yl)-1,2,4-triazolo[4,3-b]pyridazine, 3-(3-bromophenyl)-6-(piperidin-1-yl)-1,2,4-triazolo[4,3-b]pyridazine, 6-azepan-1-yl-3-(6-methoxypyridin-2-yl)-1,2,4-triazolo[4,3-b]pyridazine, 6-(4-fluoropiperidin-1-yl)-3-(6-methoxypyridin-2-yl)-1,2,4-triazolo[4,3-b]pyridazine, 6-(3-azabicyclo[3.2.1]octan-3-yl)-3-(6-methoxypyridin-2-yl)-1,2,4-triazolo[4,3-b]pyridazine, 6-(4,4-difluoropiperidin-1-yl)-3-(6-methoxypyridin-2-yl)-1,2,4-triazolo[4,3-b]pyridazine, 6-(3,3-difluoropiperidin-1-yl)-3-(6-methoxypyridin-2-yl)-1,2,4-triazolo[4,3-b]pyridazine, 6-azocan-1-yl-3-(6-bromopyridin-2-yl)-1,2,4-triazolo[4,3-b]pyridazine, and 6-(8-azabicyclo[3.2.1]octan-8-yl)-3-(6-bromopyridin-2-yl)-1,2,4-triazolo[4,3-b]pyridazine.

Depending on the substituent groups of the compound (I), it may exist as geometric isomers or tautomers, and the invention includes all of these isomers as being separated or in a mixture. The compound (I) may have an asymmetric carbon atom, based on which an optical isomer may exist. The invention includes all of these optical isomers as mixtures or individually separated forms.

The compound (I) may form an acid addition salt or a salt with a base depending on the type of the substituent group. Such a salt is a pharmaceutically acceptable salt, preferably an acid addition salt with an inorganic acid such as hydrochloric acid, hydrobromic acid, hydroiodic acid, sulfuric acid, nitric acid, phosphoric acid and the like and with an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid and the like, a salt with an inorganic base including a metal such as sodium, potassium, magnesium, calcium, aluminum or the like, and with an organic base such as methylamine, ethylamine, ethanolamine, lysine, ornithine or the like, as well as an ammonium salt.

Furthermore, the invention includes various hydrates or solvates of the compound (I) or a salt thereof, as well as the forms of polymorphic crystals.

As shown in Example 1 which is described later, the compound (I) of the invention is possessed of excellent ALP promoting activity, and has a bone formation promoting action by itself through the osteoblast differentiation induction promotion.

According to the invention, the "bisphosphonate" is a bisphosphonic acid having a bone resorption inhibitory action or a pharmaceutically acceptable salt or ester thereof, and is preferably etidronate, alendronate (JP-B-2-13645, JP-B-6-62651, US Patent 4705651), risedronate (Japanese Patent No. 2702419, Japanese Patent No. 2568999, European Patent 186405), pamidronate (JP-B-5-8717, US Patent 4327039), incadronate (JP-B-7-629), clodronate, minodronate (JP-B-6-99457), ibandronate (JP-B-8-2913, European Patent 252504), zoledronate (Japanese Patent No. 2744238, European Patent 275821), tiludronate (JP-B-4-29676, Japanese Patent No. 2735462, US Patent 5739381), neridronate (JP-B-63-7526, US Patent 4578376) or the like. More preferred is alendronate, risedronate, pamidronate, incadronate, minodronate, ibandronate or zoledronate, and particularly preferred is alendronate, risedronate, incadronate, minodronate or zoledronate.

As the pharmaceutically acceptable salt of bisphosphonic acid, salts with an inorganic base including a metal such as sodium, potassium, magnesium, calcium, aluminum or the like, and with an organic base such as methylamine, ethylamine, ethanolamine, lysine, ornithine or the like can be exemplified. Regarding its ester, it is preferably a methyl ester, an ethyl ester or the like lower alkyl ester.

Regarding these bisphosphonates, those which are on the market may be used, or these may be produced by the methods described in the aforementioned references.

In addition, the "bone mass increasing inducer" according to the invention means an agent capable of increasing the bone mass and/or bone strength, and is preferably an agent which increase both of the bone mass and bone strength. Accordingly, included in the "bone mass increasing inducer" of the invention are preventive or therapeutic agents for osteoporosis, osteitis fibrosa (hyperparathyroidism), osteomalacia, Paget's disease and the like metabolic bone diseases which accompany reduction of the bone mass and/or bone strength; bone tissue restoring agents for bone tissue damages such as bone fracture, re-bone fracture, bone injury, osteoarthropathy, multiple myeloma, bone metastasis and the like; and further, a periodontosis treating agent in the field of dentistry, a restoring agent for periodontal tissue damage, a stabilizer of the artificial root of a tooth, a jaw bank formation promoting agent and the like. As the "bone mass increasing inducer" of the invention, preferred is a preventive or therapeutic agent for osteoporosis and the like metabolic bone diseases, and particularly preferred is a preventive or therapeutic agent for a low bone turnover type (type II) osteoporosis in which the bone forming ability was reduced, especially senile osteoporosis.

According to the "combination product which is a bone mass increasing inducer" of the invention, the "combination product" means a product in which respective components are independent pharmaceutical preparations and can be used in a concomitant therapy, and this may be put for sale as a package of their combination (e.g., a form of a kit or the like) or each independently for concomitant administration. In this connection, the "simultaneously" means that the first pharmaceutical preparation and the second pharmaceutical preparation are administered at the same time, and the "separately" means that the first pharmaceutical preparation and the second pharmaceutical preparation are separately administered through the same or different route of administration and at the same or different administration frequency or administration interval. Preferably, these are simultaneously or separately administered under pharmaceutical preparation formulation, route of administration, administration frequency and the like administration conditions suited for respective pharmaceutical preparations, by taking bioavailability, stability and the like of each pharmaceutical preparation into consideration.

An example of the kit is a package which comprises a first pharmaceutical preparation containing a non-peptide osteoblast differentiation promoting compound and a second pharmaceutical preparation containing a bisphosphonate and, as occasion demands, further comprises a placebo preparation or the like additional pharmaceutical preparation and a display member, which facilitate the administration corresponding to respective administration stages.

According to the invention, the "bone mass gain reinforcing agent" means an agent which, when other agent has a function to induce bone mass gain as a result, further increases the bone mass in harmony with the function, and illustratively,
(a) regarding the bisphosphonate, since the bone mass increasing effect of an osteoblast differentiation promoting compound is reinforced by the concomitant use of the bisphosphonate, it means an agent which reinforces the bone mass increasing effect of the osteoblast differentiation promoting compound, and on the other hand,
(b) regarding the osteoblast differentiation promoting compound, since the bone mass increasing effect of the bisphosphonate is reinforced by the concomitant use of the osteoblast differentiation promoting compound, it means an agent which reinforces the bone mass increasing effect of the bisphosphonate.

A pharmaceutical composition comprising the non-living body-derived non-peptide osteoblast differentiation promoting compound of the invention and a bisphosphonate, and the aforementioned first pharmaceutical preparation and the second pharmaceutical preparation constituting the combination product of the invention, can be respectively prepared using ordinary methods well known to those skilled in the art. That is, these can be prepared by generally used methods using a pharmaceutical carrier, filler and other additives employed usually in a formulation. The administration may be conducted by either an oral administration through tablets, pills, capsules, granules, powders, liquids, inhalations and the like, or a parenteral administration through injections such as an intravenous injection, intramuscular injection and the like, as well as suppositories, percutaneous liquid formulations; ointments, percutaneous patches and the like.

As a solid composition of the invention for oral administration, tablets, powders, granules and the like are used. In such a solid composition, one or more active substances are mixed with at least one inert diluent such as lactose, mannitol, glucose, hydroxypropyl cellulose, microcrystalline cellulose, starch, polyvinyl pyrrolidone, magnesium aluminate metasilicate and the like. In accordance with the usual way, the composition may contain additives other than the inert diluent including a lubricant such as magnesium stearate, a disintegrator such as calcium fibrinoglycolate, a stabilizer, and a solubilizing agent such as glutamic acid or aspartic acid. A tablet or pill may be sugar-coated as occasion demands with sucrose, gelatin, hydroxypropyl cellulose, hydroxypropylmethyl cellulose phthalate and the like or covered with a gastric or enteric film coating.

A liquid composition for an oral administration includes pharmaceutically acceptable emulsions, solutions, suspensions, syrups, elixirs and contains an ordinarily employed inert diluent such as purified water and ethanol. Such a composition may contain, in addition to an inert diluent, other auxiliary agents such as humectants and suspending agents, as well as a sweetener, flavor, aromatic and preservative.

An injection formulation for a parenteral administration includes an aseptic aqueous or non-aqueous solution, suspension and emulsion. Such an aqueous solution and suspension may contain a distilled water for injection or a physiological saline. A non-aqueous solution and suspension may contain propylene glycol, polyethylene glycol, a vegetable oil such as an olive oil, an alcohol such as ethanol, Polysorbate 80 (trade name) and the like. Such a composition may also contain auxiliary agents such as a preservative, humectant, emulsifier, dispersing agent, stabilizer, solubilizing agent (e.g., glutamic acid or aspartic acid) and the like. Any of these materials can be made aseptic by filtration through a bacteria retaining filter, incorporation of a sterilizing agent or irradiation. Any of these material can be formulated as an aseptic solid composition which is to be reconstituted just before use with an aseptic water or aseptic solvent for injection.

In addition, various techniques regarding bisphosphonate-containing pharmaceutical preparations are disclosed in International Publication 94/05297, International Publication 99/04773, US Patent 5358941, International Publication 99/18972, International Publication 97/44017, International Publication 2000/21541, International Publication 2001/82903, International Publication 2001/76577 and the like, and a desired bisphosphonate-containing pharmaceutical preparation can also be produced by optionally applying these conventionally known techniques.

The non-living body-derived non-peptide osteoblast differentiation promoting compound to be used in the pharmaceutical composition or combination product of the invention is orally or parenterally administered at a dose which is effective in yielding the osteoblast differentiation promoting activity. When a non-living body-derived non-peptide osteoblast differentiation promoting compound conventionally known by the aforementioned references is used, the suitable dosage and administration form described in said references can be employed. For example, when the compound (I) of the invention is used, its daily dose in the case of oral administration is approximately from 0.001 to 100 mg/kg body weight, preferably from 0.01 to 60 mg/kg, and this is administered once or by dividing into from 2 to 4 doses. Alternatively, the doses for several days to several months may be combined and administered at a frequency of, for example, once a week, twice a week, twice a month, once a month or the like. When intravenously administered, approximately from 0.0001 to 20 mg/kg body weight, preferably from about 0.001 to 5 mg/kg, is appropriate, and this is administered once a day or by dividing into two or more doses. Alternatively, the doses for several days to several months may be combined and administered at a frequency of, for example, once a week, twice a week, twice a month, once a month or the like. The non-living body-derived non-peptide osteoblast differentiation promoting compound of the invention may be topically administered as a topical injection preparation or a depot preparation to a region where bone formation is desired.

On the other hand, the bisphosphonate to be used in the pharmaceutical composition or combination product of the invention is orally or parenterally administered at a dose which is effective in yielding the bone resorption inhibitory activity. Daily dose of the bisphosphonate is decided in response to the activity of the bisphosphonate to be used. In the case of a bisphosphonate having conventionally known dose and administration frequency suited for clinical use, it is desirable to administer it at said clinical dose and administration frequency. Alternatively, it may be administered at a smaller dose than that, by taking its synergistic effect with the osteoblast differentiation promoting compound into consideration. For example, in the case of the oral administration of alendronate, risedronate, pamidronate, incadronate, minodronate, ibandronate or zoledronate, approximately from 0.001 to 30 mg per day, preferably from 0.01 to 10 mg, is appropriate, and this is administered once a day, or by combining the doses for several days to several months and administering he mixture at a frequency of, for example, once a week, twice a week, twice a month, once a month or the like. When intravenously administered, approximately from 0.0001 to 1 mg/kg body weight, preferably from 0.001 to 0.5 mg/kg, is appropriate, and this is administered once a day, or by combining the doses for several days to several months and administering he mixture at a frequency of, for example, once a week, twice a week, once a month, once in 3 months, once in 6 months, once in a year or the like.

In the pharmaceutical composition of the invention which comprises the non-living body-derived non-peptide osteoblast differentiation promoting compound and a bisphosphonate, both components are prepared and produced in such a manner that the amounts corresponding to the aforementioned respective doses are included.

Since the compound (I) to be used in the invention is a novel compound, its typical production methods are described in the following.

The compound (I) and a salt thereof can be produced by employing various conventionally known synthesis methods, making use of characteristics based on its basic skeleton or kinds of substituent groups. In that case, depending on the kind of functional group, it is sometimes effective in view of the production techniques to substitute said functional group with an appropriate protecting group, namely a group which can be easily converted into said functional group, at the stage of the material or an intermediate. Thereafter, the desired compound can be obtained by removing the protecting group in response to the necessity. As such a functional group, hydroxyl group, carboxyl group and the like can for example be cited, and as their protecting groups, the protecting groups described in "Protective Groups in Organic Synthesis", second edition, edited by Greene and Wuts, which may be optionally used in response to the reaction conditions.

Typical production methods are described below.

### First production method

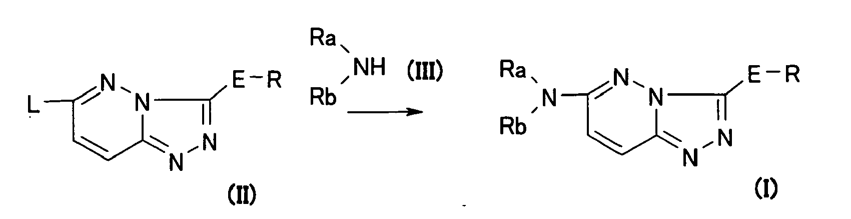

(In the reaction scheme, L is a generally used leaving group such as a halogen atom or an organic sulfonate. The same applies analogously to the followings.)

The compound (I) can be obtained by a standard N-alkylation method, for example by allowing an amine derivative (III) to react with a compound (II) having an ordinary leaving group such as a halogen atom or an organic sulfonate in the presence or absence of a base such as potassium carbonate, triethylamine, sodium hydride or the like, in an inert solvent such as *N*,*N*-dimethylformamide (DMF), toluene, tetrahydrofuran (THF), acetonitrile or the like or without using any solvent with cooling or under reflux.

### Other production method

The compound obtained in the first production method described above can further be subjected to a standard substituent-modifying reaction, for example, reduction from a nitro group to an amino group, amidation, sulfonamidation, *N*-alkylation, esterification, ester hydrolysis, hydroxyl group etherification, thioether sulfonation, halogenation, olefin-derivatization, or the like to obtain the compound of the invention having a desired substituent group. Any of these reactions can readily be conducted in accordance with a method described for example in ORGANIC FUNCTIONAL GROUP PREPARATIONS Second Edition (edited by Sandler, Karo).

### (Method for producing material compound of the compound (I))

### Production method a

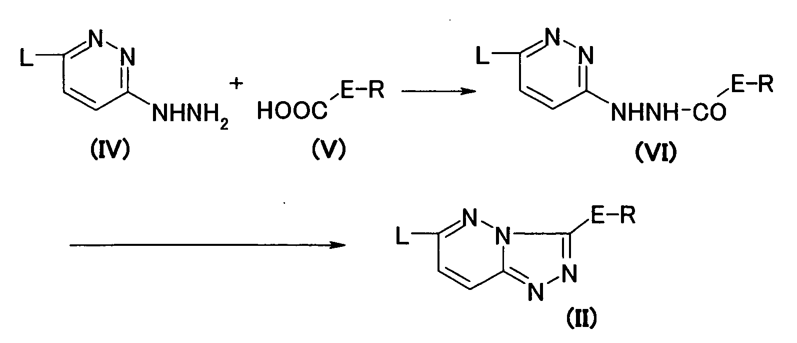

A material compound (II) can be produced by subjecting a hydrazine compound (IV) and a carboxylic acid compound (V) to a dehydration condensation reaction to form a hydrazide compound (VI) followed by a cyclization.

The dehydration condensation reaction in the first step can be easily carried out by a standard method, for example by using a free carboxylic acid and a coupling agent such as 1-(3-dimethylaminopropyl)-3-ethylcarbodiimide (WSCD) or a carboxylic acid-activating agent such as 1,1'-carbonyldiimidazole, or using a reactive derivative of a carboxylic acid (e.g., an acid halide such as acid chloride, acid bromide or the like; an acid azide; an active ester which can be prepared using methanol, ethanol, benzyl alcohol, optionally substituted phenol, N-hydroxysuccinimide or the like; a symmetric acid anhydride; a mixed acid anhydride with an alkylcarbonate, p-toluenesulfonic acid or the like).

The reaction is carried out using equimolar amounts or an excessive amount of any one of the reactants, in an organic solvent which is inert to the reaction, such as pyridine, THF, methylene chloride, DMF, acetonitrile or the like. The reaction temperature is selected appropriately depending on the type of the reactive derivative. In a case of a certain reactive derivative, it may be advantageous to add a base such as 4-dimetylaminopyridine for promoting the reaction.

The cyclization reaction in the second step can be carried out by a reaction in the presence or absence of an acid such as acetic acid, p-toluenesulfonic acid, hydrochloric acid or the like, in a solvent such as xylene, ethylene glycol or the like, or without using any solvent. This reaction can be carried out at room temperature or with heating under reflux.

### Production method b

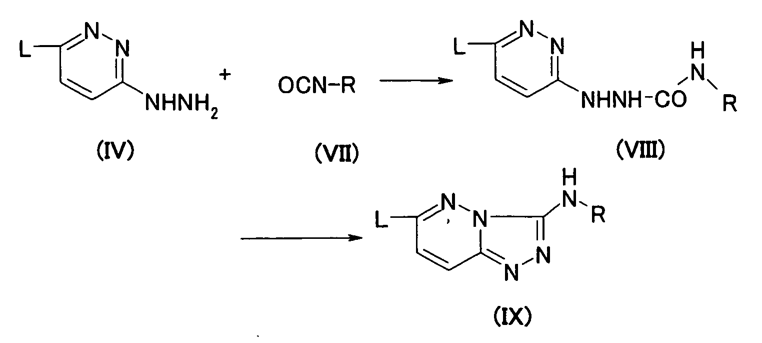

A material compound (IX) can be produced by coupling a compound (IV) with an isocyanate compound (VII) in a solvent inert to the reaction such as acetonitrile to form a compound (VIII) followed by adding 1,2-dibromo-1,1,2,2-tetrachloroethane and triphenylphosphine in the presence of a base such as triethylamine whereby effecting a cyclization. The reaction can be carried out appropriately by a standard method at room temperature or with heating under reflux.

A reaction product obtained by each production method described above can be isolated and purified as a free compound, its salt, hydrate or any of various solvates. A salt can be produced by an ordinary salt formation reaction. The isolation and the purification can be carried out by applying ordinary chemical procedures such as extraction, concentration, distillation, crystallization, filtration, recrystallization, various chromatographic means and the like. Each isomer can be isolated utilizing a physicochemical difference between isomers. For example, an optical isomer can be separated by an ordinary optical resolution method, for example, fractional crystallization or chromatography. In addition, an optical isomer can also be synthesized from a suitable optically active material compound.

Next, the invention is described further in detail based on examples, though the invention is not limited to these examples.

### Example 1: Alkaline phosphatase (ALP) activity of compound (I)

A mouse osteoblastic cell line MC3T3-E1 in fetal bovine serum (FBS)-containing α-minimum essential medium (MEM) was seeded in 96 well plates at the density of 3000 cells/well and cultured for 4 to 6 hours. Each of the test compounds dissolved in dimethyl sulfoxide (DMSO) was added to the thus adhered cells (DMSO final concentration 0.5%), and the culturing was continued for 3 days. The cells were washed with phosphate buffered physiological saline and then mixed with a substrate and incubated at 37°C for 10 to 15 minutes. The reaction was stopped by adding 0.5 M sodium hydroxide and the absorbance at 405 nm (reference wavelength 492 nm) was measured to calculate the ALP activity as a % value based on the control group which was set to 100%. In this connection, the aforementioned measuring method was carried out by referring to the method of Lowry et al. (*Journal of Biological Chemistry,* vol. 207, p. 19 (1954)).

(Results) Many compounds shown in the following Production Examples 2, 6, 10, 19, 21, 22, 25, 26, 31, 32, 33, 36, 37, 40, 43, 52, 63, 65, 70, 73, 74, 79, 82, 83, 84, 85, 90, 91, 92, 93, 94, 98, 101, 105, 112, 114, 115 and 116, which are included in the compound (I), were possessed of 300% or more of the ALP activity based on the control group at a concentration of 300 nM. Accordingly, it was confirmed that the compound (I) of the invention promotes the ALP activity and has a good osteoblast differentiation promoting activity.

### Example 2: Test using an osteoporosis model (rat OVX)

### <Preparation of osteoporosis model>

Female SD rats of 16 weeks of age (Charles River Japan) were used in the test. Under anesthesia, both sides of ovaries were exposed, and ovaries of all groups excluding the sham operation group were ligated and extracted. In the sham operation group, only an operation of exposing ovaries to the outside of abdomen was carried out.

Preparation of the aforementioned model was carried out by referring to the method of Kalu *et al*., (*Bone Miner*., vol. 15, p. 175 (1991)) and the method of Frost *et al*., (*Bone Miner.*, vol. 18, p. 227 (1992)).

### <Test 1>

### 1) Test method

Before carrying out the ovariectomy (OVX), bone mineral density at 5 mm from the tibial proximal end was measured by pQCT. By leaving for 8 weeks after the OVX, significant reduction of the bone mineral density was confirmed. The animals were divided into groups in such a manner that bone mineral densities of respective groups do not have significant difference, and then each compound to be tested was repeatedly administered for 12 weeks. The bone density was measured every 4 weeks after the administration, and urine samples were collected during 24 hours from the previous day of the completion of observation period. After completion of the urine collection, they were sacrificed by exsanguinations under ether anesthesia, and samples were collected.

### (a) Femoral bone strength (three-point bending test) measuring method

Bone strength of the femoral diaphysis was measured by three-point bending test (TK-252c, mfd. by Muromachi Kikai). A femur was gently put on a supporting table of 20 mm in distance between supporting points, a bending load was applied on the midpoint of femur at a rate of 10 mm/min, and a load-deformation curve was recorded until its breakage. Maximum value of the load-deformation curve was used as the ultimate force, and the slope of the linear portion of the load-defomation curve was used as the stiffness.

### (b) Non-invasive measuring method (pQCT) of bone mineral density and the like various parameters

Using a peripheral quantitative computed tomography (pQCT, XCT960A, mfd. by Norland-Stratec), cross-section of the bone was scaned, and bone mineral density and the like various parameters were calculated by analyzing the image. (c) Measuring method of urinary deoxypyridinoline value

As a bone metabolism marker, measurement of lysylpyridinoline (deoxypyridinoline hereinafter) value was carried out by SRL MediSearch using an HPLC method (Uebelhart, D *et al*., *Bone Miner*., vol. 8, p. 87 (1990)). In order to exclude influence of urine volume, the thus obtained deoxypyridinoline value was corrected by dividing the value by urine creatinine concentration or multiplying the 24 hour urine volume.

### 2) Compounds to be tested

The compound of Production Example 91 (6-(4-fluoropiperidin-1-yl)-3-(6-methoxypyridin-2-yl)-1,2,4-triazolo[4,3-b]pyridazine: to be referred to as compound A hereinafter) which is a typical compound of the compound (I) was used as the non-living body-derived non-peptide osteoblast differentiation promoting compound of the invention, and incadronate (Bisphonal (registered trademark)) as the bisphosphonate, respectively. Groups in which the compound A or incadronate alone and a parathyroid hormone hPTH(1-34) are to be administered were used as comparative groups. In addition, the drug-non-administered OVX group and the sham operation group were used as control groups. The compound A was orally administered twice a day as a 0.5% methyl cellulose suspension of 30 mg/5 ml/kg, and incadronate was orally administered once a day as an aqueous solution of 1 mg/2.5 ml/kg. The hPTH(1-34) was subcutaneously administered once a day as a physiological saline solution of 3 µg/1 ml/kg. To the control groups was orally administered 0.5% methyl cellulose twice a day.

Details of respective administration groups are shown in the following table.

**Table 1**

| Administration group | Operation | Administered compound | Dose (/kg) | Administration method |
|---|---|---|---|---|
| Concomitant group of the Present Invention | OVX | Compound A | 30 mg | Twice a day, 6 days a week, oral |
| | | Incadronate | 1 mg | Once a day, 6 days a week, oral |
| Comparative group 1 | OVX | Compound A | 30 mg | Twice a day, 6 days a week, oral |
| Comparative group 2 | OVX | Incadronate | 1 mg | Once a day, 6 days a week, oral |
| Comparative group 3 | OVX | hPTH(1-34) | 3 µg | Once a day, 5 days a week, subcutaneous |
| Control group 1 | OVX | 0.5% MC | - | Twice a day, 6 days a week, oral |
| Control group 2 | sham | 0.5% MC | - | Twice a day, 6 days a week, oral |

### 3) Results

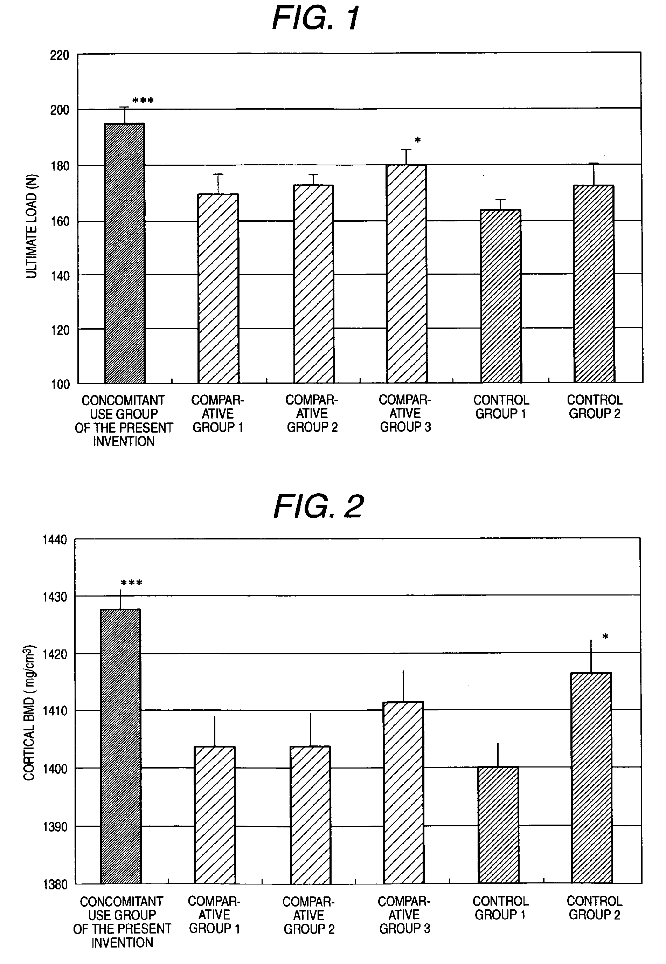

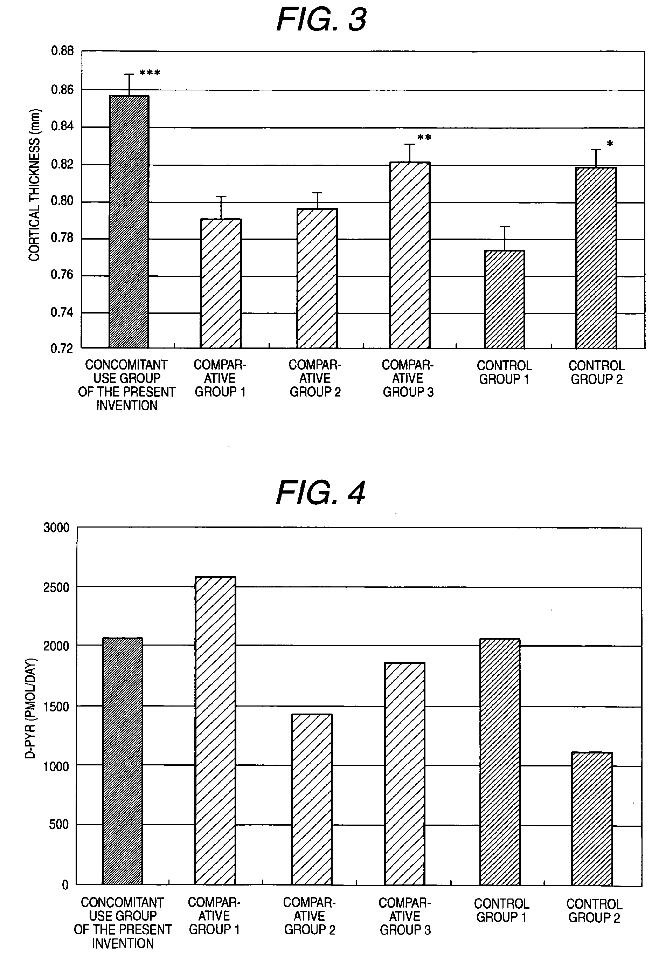

A result of the femur bone strength (three-point bending test) is shown in Fig. 1, and a result of the femoral cortical bone mineral density (pQCT) in Fig. 2 and a result of the femoral cortical bone thickness (pQCT) in Fig. 3. In addition, the urinary deoxypyridinoline value (urine volume correction) is shown in Fig. 4.

### 4) Discussion

Regarding the effects of the single administration groups of the compound A as a osteoblast differentiation promoting compound and the incadronate as a bisphosphonate (Comparative Groups 1 and 2) on the femoral cortical bone mineral density, thickness and strength, the bone mineral density, thickness and strength showed a increasing tendency in comparison with Control Group 1, but the efficacy was not sufficient. On the other hand, the femoral cortical bone mineral density, thickness and strength were markedly increased in the concomitant use group of the invention, and the effect was superior to those of the PTH (Comparative Group 3) and sham operation (Control Group 2) (cf. Figs. 1, 2 and 3 which are shown later) .

### <Test 2>

### 1) Test method

Before carrying out the ovariectomy (OVX), bone mineral density of tibial metaphysis at 5 mm from the tibial proximal end was measured by pQCT. By leaving for 8 weeks after the OVX, significant reduction of the bone mineral density of tibial metaphysis was confirmed by remeasurement. By measuring cortical bone thickness of tibial diaphysis at 15 mm from the tibial proximal end by pQCT, the animals were divided into groups in such a manner that bone mineral density of tibial metaphysis and cortical bone thickness of tibial diaphysis of each group does not have significant difference, and then each compound to be tested was repeatedly administered for 4 weeks. After 4 weeks of the administration, the cortical bone thickness and bone mineral density of the tibial diaphysis were measured in the same manner as described in the above. In addition, urine samples were collected during 24 hours 5 weeks after the administration, and the urinary deoxypyridinoline was measured by an EIA method (Osteolinks "DPD", mfd. by Sumitomo Pharmaceutical Biomedical).

### 2) Compounds to be tested

The compound A was orally administered twice a day as a 0.5% methyl cellulose suspension of 30 mg/5 ml/kg, and alendronate was orally administered once a day as an aqueous solution of 3 mg/2.5 ml/kg. To the control group was orally administered 0.5% methyl cellulose twice a day, and water once a day.

Details of respective administration groups are shown in the following table.

**Table 2**

| Administration group | Operation | Administered compound | Dose (/kg) | Administration method |
|---|---|---|---|---|
| Concomitant group of the Present Invention | OVX | Compound A | 10 mg | Twice a day, 6 days a week, oral |
| | | Alendronate | 3 mg | Once a day, 6 days a week, oral |
| Concomitant group of the Present Invention | OVX | Compound A | 30 mg | Twice a day, 6 days a week, oral |
| | | Alendronate | 3 mg | Once a day, 6 days a week, oral |
| Comparative Comparative group 1 | OVX | Compound A | 30 mg | Twice a day, 6 days a week, oral |
| | | Water | - | Once a day, 6 days a week, oral |
| Comparative group 2 | OVX | 0.5% MC | - | Twice a day, 6 days a week, oral |
| | | Alendronate | 3 mg | Once a day, 6 days a week, oral |
| Control group 1 | OVX | 0.5% MC | - | Twice a day, 6 days a week, oral |
| | | Water | - | Once a day, 6 days a week, oral |

### 3) Results

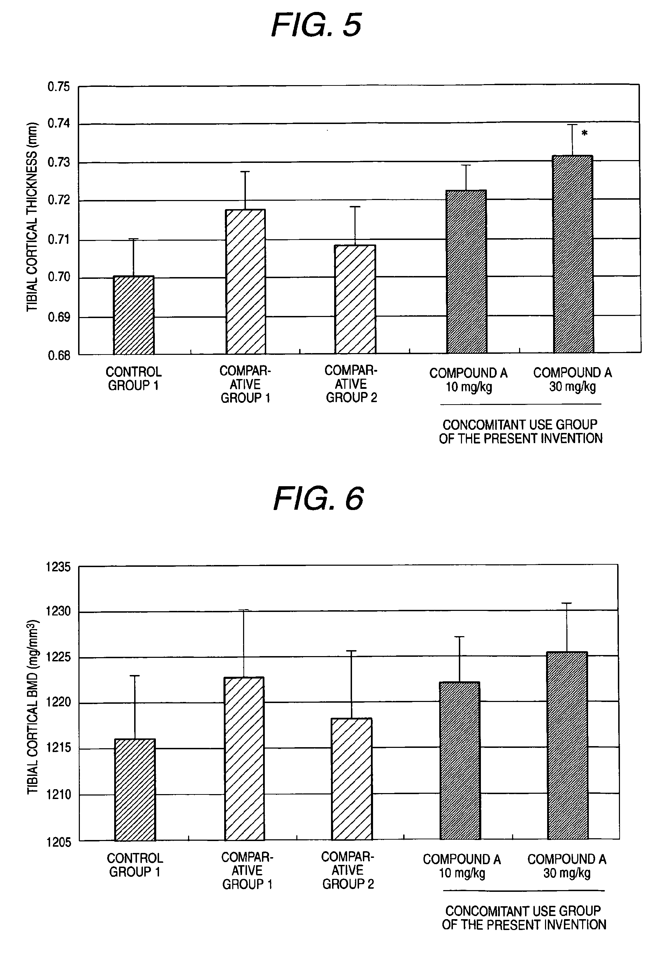

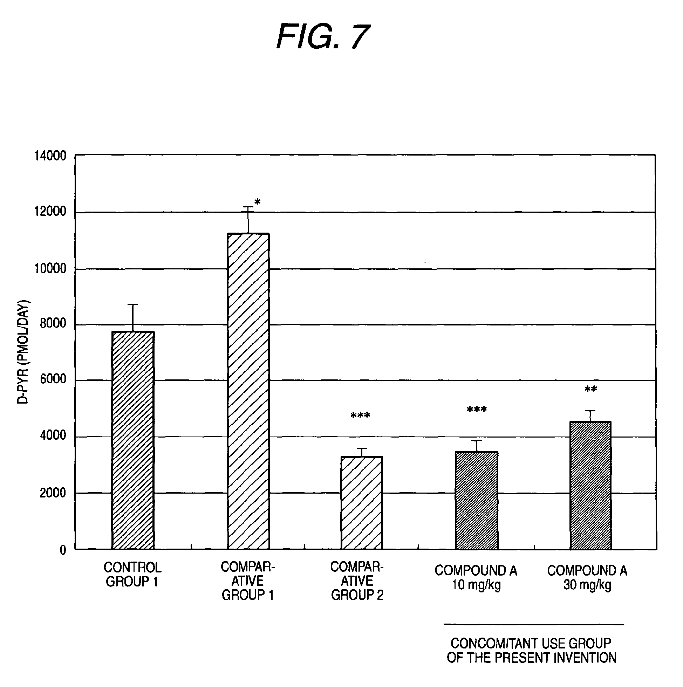

The tibial cortical bone thickness (pQCT) is shown in Fig. 5, and the tibial cortical bone density (pQCT) in Fig. 6 and the urinary deoxypyridinoline value (urine volume correction) in Fig. 7.

### 4) Discussion

In the concomitant use group of the invention, the tibial cortical bone thickness was significantly increased by the administration of merely 4 weeks, showing that the bone mass was properly increased in comparison with Comparative Groups 1 and 2.

The significant effect in the concomitant use group of the invention on the femoral or tibial cortical bone density, thickness and strength far exceeded the additive effect of each of the single administration groups (Comparative Groups 1 and 2). This was considered to be due to the synergistic or more than a simple additive increase of the bone mass and bone strength effected by proper control of the whole bone metabolism through the synergistic action of the bone metabolism turnover acceleration effect of the non-living body-derived non-peptide osteoblast differentiation promoting compound and the bone resorption inhibitory effect of the bisphosphonate. Since the preventive effect of the current therapeutic methods which use a bisphosphonate and PTH is said to be insufficient for a clinically serious bone fracture of femoral neck (cortical bone), it is considered that the other concomitant drug of the invention capable of markedly and particularly increasing the cortical bone strength is clinically markedly useful.

Since the compound A having good in vitro osteoblast differentiation promoting action increased a bone resorption index, urinary deoxypyridinoline value, by the administration of compound A alone (cf. Fig. 4 and Fig. 7), it is considered that it also increased the bone resorption activity. That is, since the osteoblast differentiation promoting compound accelerates bone formation, but also accelerates bone resorption at the same time, and thereby accelerates the whole bone metabolism turnover, it was considered that the bone mass and/or bone strength reinforcing effect was not sufficient as the result. On the other hand, it seemed that acceleration of bone resorption was inhibited in the concomitant administration group of the invention due to reduction of the urinary deoxypyridinoline value (cf. Fig. 4 and Fig. 7), it was considered that the aforementioned significant synergistic effect was obtained as a result that the bisphosphonate did not inhibit bone formation acceleration action of the osteoblast differentiation promoting compound, but inhibited the bone resorption acceleration alone.

### Example 3: Production examples of compound (I)

### (Examples)

Illustrative production examples of the compound (I) and reference examples as production examples of the material compounds are described in the following. In this connection, abbreviations in the text are Dat: physicochemical characteristics, F: FAB-MS (M+H)⁺; other symbols are as defined in the foregoing.

Reference Example 1: A mixture of (6-chloropyridazin-3-yl)hydrazine, 4-nitrobenzoic acid, WSCD hydrochloride and THF was stirred at room temperature for 2 hours. The reaction solution was combined with water, and the precipitate was collected by filtration, washed with water and diethyl ether to obtain *N*'-(6-chloropyridazin-3-yl)-4-nitrobenzohydrazide. To this was added acetic acid, and the mixture was stirred at 110°C for 2 hours, the reaction solution was concentrated under a reduced pressure, and the resultant crude crystals were washed with ethanol to obtain 6-chloro-3-(4-nitrophenyl)-1,2,4-triazolo[4,3-b]pyridazine. Dat (F:276).

Reference Example 2: To a solution of (6-chloropyridazin-3-yl)hydrazine and triethylamine in THF, 3-cyanobenzoyl chloride was added with cooling in ice, and stirred at room temperature for 1 hour. The reaction solution was combined with water, and the precipitate was collected by filtration and washed with water and diethyl ether to obtain *N*'-(6-chloropyridazin-3-yl)-3-cytanobenzohydrazide. To this, acetic acid was added, and the mixture was stirred at 110°C for 2 hours, and then the reaction solution was concentrated under reduced pressure, and the resultant crude crystals were washed with ethanol to obtain 3-(6-chloro-1,2,4-triazolo[4,3-b]pyridazin-3-yl)benzonitrile. Dat (F:256).

Reference Example 3: A mixture of (6-chloropyridazin-3-yl)hydrazine, 3-dimethylaminobenzoic acid, WSCD hydrochloride and THF was stirred at room temperature for 2 hours. The reaction solution was combined with water, and the precipitate was collected by filtration and washed with water and isopropyl ether to obtain *N*'-(6-chloropyridazin-3-yl)-3-dimethylaminobenzohydrazide. To this, ethylene glycol was added, and the mixture was stirred at 160°C for 4 hours. After allowing to cool to room temperature, followed by purification by a standard method, 6-chloro-3-(3-dimethylaminophenyl)-1,2,4-triazolo[4,3-b]pyridazine was obtained. Dat (F:274).

Reference Example 4: A solution of 3-methoxyphenyl isocyanate and 3-chloro-6-hydrazinopyridazine in acetonitrile was stirred at room temperature for 30 minutes, and then 1,2-dibromo-1,1,2,2-tetrachloroethane was added thereto and the mixture was stirred at room temperature for further 2 hours. This reaction solution was combined with triethylamine and triphenylphosphine with cooling in ice and stirred at room temperature for 3 days, and then the reaction solution was concentrated under reduced pressure and purified by a standard method to obtain 6-chloro-*N*-(3-methoxyphenyl)-1,2,4-triazolo[4,3-b]pyridazin-3-amine. Dat (F:276).

Similarly to Reference Example 1, the compounds of Reference Examples 5 to 50 indicated in Tables 3 and 4 shown below were obtained.

Production Example 1: A mixture of 3-(6-chloro-1,2,4-triazolo[4,3-b]pyridazin-3-yl)benzonitrile (450 mg) and piperidine (5 ml) was stirred with heating under reflux for 2 hours. The reaction solution was concentrated under a reduced pressure, and the resultant residue was extracted with chloroform. The extract was washed with saturated aqueous solution of ammonium chloride and saturated brine, dried over anhydrous magnesium sulfate, and then concentrated under a reduced pressure. The resultant crude crystals were recrystallized from ethanol to obtain 3-[6-(piperidin-1-yl)-1,2,4-triazolo[4,3-b]pyridazin-3-yl)benzonitrile (435 mg) as slightly yellowish crystals.

Production Example 2: To a solution of N-cyclopentyl-3-(3-methoxyphenyl)-1,2,4-triazolo[4,3-b]pyridazine-6-amine (300 mg) in DMF (5 ml), 60% sodium hydride (44 mg) was added with cooling in ice, and the mixture was stirred at an ice-cooling temperature to room temperature for 1 hour, combined with methyl iodide (68 µl), and stirred further for 2 hours. The reaction mixture was combined with water and then extracted with ethyl acetate. The extract was washed with saturated brine, dried over anhydrous magnesium sulfate, and then concentrated under a reduced pressure. The resultant residue was purified by a silica gel column chromatography (eluent: chloroform:methanol = 20:1) and then recrystallized from ethyl acetate to obtain *N-*cyclopentyl-3-(3-methoxyphenyl)-*N*-methyl-1,2,4-triazoro[4,3-b]pyridazin-6-ylamine (145 mg) as pale yellow crystals.

Production Example 3: To a mixture of potassium hydroxide (98 mg) and DMSO (5 ml), 6-azepan-1-yl-3-(1H-indol-2-yl)-1,2,4-triazolo[4,3-b]pyridazine (501 mg) was added, and the mixture was stirred at room temperature for 30 minutes, combined with methyl iodide (0.15 ml) and then stirred for further 2 hours at room temperature. The reaction mixture was combined with water, and the resultant solids were collected by filtration, washed with a mixed solvent of water and methanol, and then purified by a silica gel column chromatography (eluent: chloroform: methanol = 99:1). The resultant crude crystals were recrystallized from ethanol to obtain 6-azepan-1-yl-3-(1-methylindol-2-yl)-1,2,4-triazolo[4,3-b]pyridazine (40 mg) as colorless crystals.

Production Example 4: A mixture of 3-[6-(piperidin-1-yl)-1,2,4-triazolo[4,3-b]pyridazin-3-yl]aniline (180 mg) and acetic anhydride (3 ml) was stirred at room temperature for 6 hours. The reaction solution was concentrated under a reduced pressure, and the resultant crude crystals were washed with diethyl ether to obtain 3'-[6-(piperidin-1-yl)-1,2,4-triazolo[4,3-b]pyridazin-3-yl]acetoanilide (190 mg) as colorless crystals.

Production Example 5: To a mixed solution of 6-piperidin-1-yl-3-piperidin-3-yl-1,2,4-triazolo[4,3-b]pyridazine (670 mg), triethylamine (360 mg) and methylene chloride (15 ml), methanesulfonyl chloride (320 mg) was added and the mixture was stirred at room temperature for 8 hours. The reaction solution was concentrated under a reduced pressure, and the resultant residue was extracted with ethyl acetate. The extract was washed successively with water and saturated brine, dried over anhydrous sodium sulfate, and concentrated under a reduced pressure. The resultant crude crystals were recrystallized from ethanol to obtain 3-(1-methanesulfonylpiperidin-3-yl)-6-piperidin-1-yl-1,2,4-triazolo[4,3-b]pyridazine (230 mg) as colorless crystals.

Production Example 6: A mixture of 6-azocan-1-yl-3-(6-chloropyridin-2-yl)-1,2,4-triazolo[4,3-b]pyridazine (360 mg), sodium methoxide (570 mg) and toluene (20 ml) was stirred with heating under reflux for 3 hours. After allowing to cool to room temperature, the reaction solution was concentrated under a reduced pressure, and the resultant residue was extracted with ethyl acetate. The extract was washed with water and saturated brine, dried over anhydrous magnesium sulfate, and then concentrated under a reduced pressure. The resultant crude crystals were washed with diethyl ether to obtain 6-azocan-1-yl-3-(6-methoxypyridin-2-yl)-1,2,4-triazolo[4,3-b]pyridazine (290 mg) as pale yellow crystals.

Production Example 7: To a mixed solution of 3-[6-(piperidin-1-yl)-1,2,4-triazolo[4,3-b]pyridazin-3-yl]benzoic acid (430 mg), a catalytically effective amount of DMF and THF (10 ml), oxalyl chloride (0.44 ml) was added with cooling in ice, and the mixture was stirred at room temperature for 2 hours and then ammonia gas was passed with cooling in ice for 15 minutes. The reaction solution was concentrated under a reduced pressure, and the resultant residue was combined with chloroform:methanol (10:1), and then the insoluble matter was removed by filtration. The filtrate was concentrated under a reduced pressure, and the resultant crude crystals were recrystallized from ethanol to obtain 3-[6-(piperidin-1-yl)-1,2,4-triazolo[4,3-b]pyridazin-3-yl]benzamide (242 mg) as slightly tan crystals.

Production Example 8: To a solution of 3-[3-(methylsulfanyl)phenyl]-6-piperidin-1-yl-1,2,4-triazolo[4,3-b]pyridazine (550 mg) in methylene chloride (30 ml), 3-chloroperbenzoic acid (1.25 g) was added at room temperature, and the mixture was stirred for 13 hours. The reaction solution was combined with water and diluted with methylene chloride. The organic phase washed successively with water, 1 M aqueous solution of sodium hydroxide and saturated brine, dried over anhydrous sodium sulfate, and then concentrated under a reduced pressure. The resultant residue was purified by a silica gel column chromatography (eluent: chloroform:methanol = 98:2) and then washed with ethanol to obtain 3-[3-(methylsulfonyl)phenyl]-6-piperidin-1-yl-1,2,4-triazolo[4,3-b]pyridazine (250 mg) as colorless crystals.

Production Example 9: A mixed solution of concentrated sulfuric acid (1.5 ml) and water (3 ml) was cooled to -5°C, and 4-(6-piperidin-1-yl-1,2,4-triazolo[4,3-b]pyridazin-3-yl)-1,3-thiazole-2-amine (0.90 g), copper (II) sulfate (1.50 g) and sodium bromide (0.62 g) were added successively thereto, and the mixture was stirred at 0°C for 5 minutes, and then, a solution of sodium nitrite (0.25 g) in water (1.6 ml) was added dropwise thereto, followed by stirring at room temperature overnight. The reaction solution was combined with water, chloroform and 2-propanol, and the insoluble matter was removed by filtration. The resultant organic layer was washed with water, dried over anhydrous magnesium sulfate, and then concentrated under a reduced pressure. The resultant residue was purified by a silica gel column chromatography (eluent: chloroform:methanol = 20:1), and the thus obtained crude crystals were recrystallized from ethanol-diethyl ether to obtain 3-(2-bromo-1,3-thiazol-4-yl)-6-piperidin-1-yl-1,2,4-triazolo[4,3-b]pyridazine (75 mg) as pale yellow crystals.

Production Example 10: A mixture of 6-(6-azepan-1-yl-1,2,4-triazolo[4,3-b]pyridazin-3-yl)pyridine-2-ol (700 mg) and phosphorus tribromide (7 ml) was stirred at 130°C for 6 hours. After allowing to cool to room temperature, an ice-water was added thereto, and the mixture was neutralized with saturated aqueous solution of potassium carbonate and extracted with chloroform. The extract was washed with saturated aqueous solution of sodium bicarbonate and saturated brine, dried over anhydrous sodium sulfate, and then concentrated under a reduced pressure. The resultant residue was purified by a silica gel column chromatography (eluent: chloroform:methanol = 98:2) and then recrystallized from ethanol to obtain 6-azepan-1-yl-3-(6-bromopyridin-2-yl)-1,2,4-triazolo[4,3-b]pyridazine (240 mg) as colorless crystals.

Production Example 11: To a solution of diethyl [(6-azepan-1-yl-1,2,4-triazolo[4,3-b]pyridazin-3-yl)methyl]phosphonate (367 mg) in THF (10 ml), potassium tert-butoxide (127 mg) was added with cooling in an ice bath, and the mixture was stirred at room temperature for 40 minutes. The resultant red solution was combined with 2-bromobenzaldehyde (0.128 ml) and stirred at room temperature for further 1 hour. The reaction solution was combined with water and extracted with ethyl acetate. The extract was washed with water and saturated brine, dried over anhydrous magnesium sulfate, and then concentrated under a reduced pressure. The resultant white solids were recrystallized from methanol to obtain 6-azepan-1-yl-3-[(*E*)-2-(2-bromophenyl)vinyl]-1,2,4-triazolo[4,3-b]pyridazine (152 mg) as colorless crystals.

Production Example 12: A mixture of ethyl 6-chloro-1,2,4-triazolo[4,3-b]pyridazine-3-carboxylate (3.00 g) and hexamethylenimine (10 ml) was stirred at 100°C for 2 hours. The reaction solution was concentrated under a reduced pressure, and the resultant residue was purified by a silica gel column chromatography (eluent: chloroform:methanol = 30:1), and the thus obtained crude crystals were recrystallized from ethanol and diethyl ether to obtain 3-(azepan-1-ylcarbonyl)-6-azepan-1-yl-1,2,4-triazolo[4,3-b]pyridazine (0.23 g) as colorless crystals.

Production Example 13: A mixture of 1-[(benzyloxy)carbonyl]piperidine-3-carboxylic acid (2.44 g), 3-chloro-6-hydrazinopyridazine (1.34g), WSCD hydrochloride (2.13 g) and methylene chloride (60 ml) was stirred at room temperature for 16 hours. The reaction solution was concentrated under a reduced pressure and extracted with ethyl acetate. The extract was washed successively with water and saturated brine, dried over anhydrous sodium sulfate, and concentrated under a reduced pressure. The resultant reside was combined with acetic acid (80 ml), stirred at 110°C for 2 days, and then the solvent was evaporated under a reduced pressure. The resultant crude crystals were washed with ethanol and stirred with heating under reflux in piperidine (10 ml) for 3 hours. The reaction solution was concentrated under a reduced pressure and purified by a silica gel column chromatography (eluent: chloroform:methanol = 97:3). The resultant colorless solids were combined with ethanol (40 ml) and 10% palladium-carbon (150 mg) and stirred under an atmosphere of hydrogen at room temperature for 6 hours, and then the catalyst was removed by filtration. The resultant filtrate was concentrated under a reduced pressure to obtain 6-piperidin-1-yl-3-piperidin-3-yl-1,2,4-triazolo[4,3-b]pyridazine (1.18 g) as a colorless amorphous substance.

Production Example 14: To a solution of ethyl 2-aminothiazole-4-carboxylate (4.56 g) in THF (200 ml), 1 M aqueous solution of sodium hydroxide (30 ml) was added and the mixture was stirred at room temperature for 3 hours. The reaction solution was combined with 1 M hydrochloric acid (30 ml), concentrated, and then the resultant residue was dissolved in DMF (50 ml). Subsequently, 6-chloropyridazin-3-ylhydrazine (3.83 g) and WSCD hydrochloride (6.09 g) were added thereto, and the mixture was stirred at room temperature. The reaction solution was combined with water, and the resultant precipitate was collected by filtration and washed with water and diethyl ether, combined with acetic acid (30 ml), heated under reflux, and then the reaction solution was concentrated under a reduced pressure. The residue was combined with saturated aqueous solution of sodium bicarbonate and extracted with ethyl acetate. The organic layer was washed with water and saturated brine, dried over anhydrous magnesium sulfate, and then concentrated under a reduced pressure. To this, piperidine (10 ml) was added and the mixture was heated at 100°C, and the reaction solution was concentrated under a reduced pressure, and the resultant residue was purified by a silica gel column chromatography (eluent: chloroform:methanol = 30:1) to obtain 3-(2-aminothiazol-4-yl)-6-(pipexidin-1-yl)-1,2,4-triazolo[4,3-b]pyridazine (0.92 g) as yellow solids.

Production Example 15: To a solution of 6-hydrazino-*N*-methyl-*N*-phenylpyridazine-3-amine (1.14 g) in methylene chloride (10 ml), 6-chloropicolic acid (0.83 g) and WSCD hydrochloride (1.22 g) were added, and the mixture was stirred at room temperature overnight. The reaction solution was purified by a silica gel column chromatography (eluent: chloroform) to obtain 6-chloro-*N'*-{6-[methyl(phenyl)amino]pyridazin-3-yl}pyridine-2-carbohydrazide (0.57 g). This compound (0.56 g) was stirred at 150°C overnight in xylene (20 ml), and the reaction solution was concentrated to obtain 3-(chloropyridin-2-yl)-*N*-methyl-*N*-phenyl-1,2,4-triazolo[4,3-b]pyridazine-6-amine (0.54 g) as colorless solids.

Production Example 16: A mixture of 6-chloro-3-(6-chloropyridin-2-yl)-1,2,4-triazolo[4,3-b]pyridazine (620 mg), heptamethylenimine (1.32 g) and 1,4-dioxane (20 ml) was stirred at 100°C for 9 hours. After allowing to cool to room temperature, the reaction solution was concentrated under a reduced pressure, and the resultant residue was extracted with ethyl acetate. The extract was washed successively with 5% aqueous solution of citric acid, water, saturated aqueous solution of sodium bicarbonate and saturated brine, dried over anhydrous sodium sulfate, and concentrated under a reduced pressure. The resultant crude crystals were recrystallized from ethanol to obtain 6-azocan-1-yl-3-(6-chloropyridin-2-yl)-1,2,4-triazolo[4,3-b]pyridazine (370 mg) as grayish white crystals.

Production Example 17: A mixture of 6-(6-chloro-1,2,4-triazolo[4,3-b]pyridazin-3-yl)pyridin-2-ol (960 mg) and piperidine (10 ml) was stirred at 100°C for 3 hours. After allowing to cool to room temperature, the reaction solution was concentrated under a reduced pressure, and the resultant crystalline residue was recrystallized from ethanol to obtain 6-(6-piperidin-1-yl-1,2,4-triazolo[4,3-b]pyridazin-3-yl)pyridin-2-ol (820 mg) as grayish white crystals.

By carrying out the operation in the same manner as in Production Example 1, and subjecting to an ordinary salt formation reaction using 4 M hydrogen chloride - ethyl acetate as occasion demands, the compounds of Production Examples 18 to 105 shown in the following Tables 6 to 12 were obtained. Also, the compound of Production Example 106 of the following Table 12 was obtained similarly to Production Example 2, and the compound of Production Example 107 similarly to Production Example 3, the compounds of Production Examples 108 and 109 similarly to Production Example 4, the compounds of Production Examples 110 to 112 of the following Tables 12 and 13 similarly to Production Example 6, the compound of Production Example 113 similarly to Production Example 8, the compounds of Examples 114 to 116 similarly to Production Example 10, the compounds of Production Examples 117 and 118 similarly to Production Example 11, the compound of Production Example 119 similarly to Production Example 16, and the compounds of Production Examples 120 and 121 similarly to Production Example 17, respectively.

The structures and the physicochemical characteristics of the compounds of Reference Examples are indicated in Tables 3 and 4 shown below, and the structures and the physicochemical characteristics of the compounds of Production Examples are indicated in Tables 5 to 13. In addition, the compounds indicated in Table 14 are examples of the compound (I) which can readily be produced using appropriate material compounds almost similarly to the methods described in Production Examples or Production Method described in the foregoing, with or without any modification obvious to those skilled in the art.

The abbreviations in Tables are Rex: Reference Example number; Ex: Production Example number; Dat: physicochemical characteristics (F: FAB-MS (M+H)⁺; M: melting point [°C]; (d): decomposition; N1: NMR (DMSO-d₆, TMS internal standard) characteristic peak δ ppm); Sal: salt (void: free base; HCl: hydrochloride; 2HCl: dihydrochloride); Me: methyl; Et: ethyl; and Ac: acetyl.

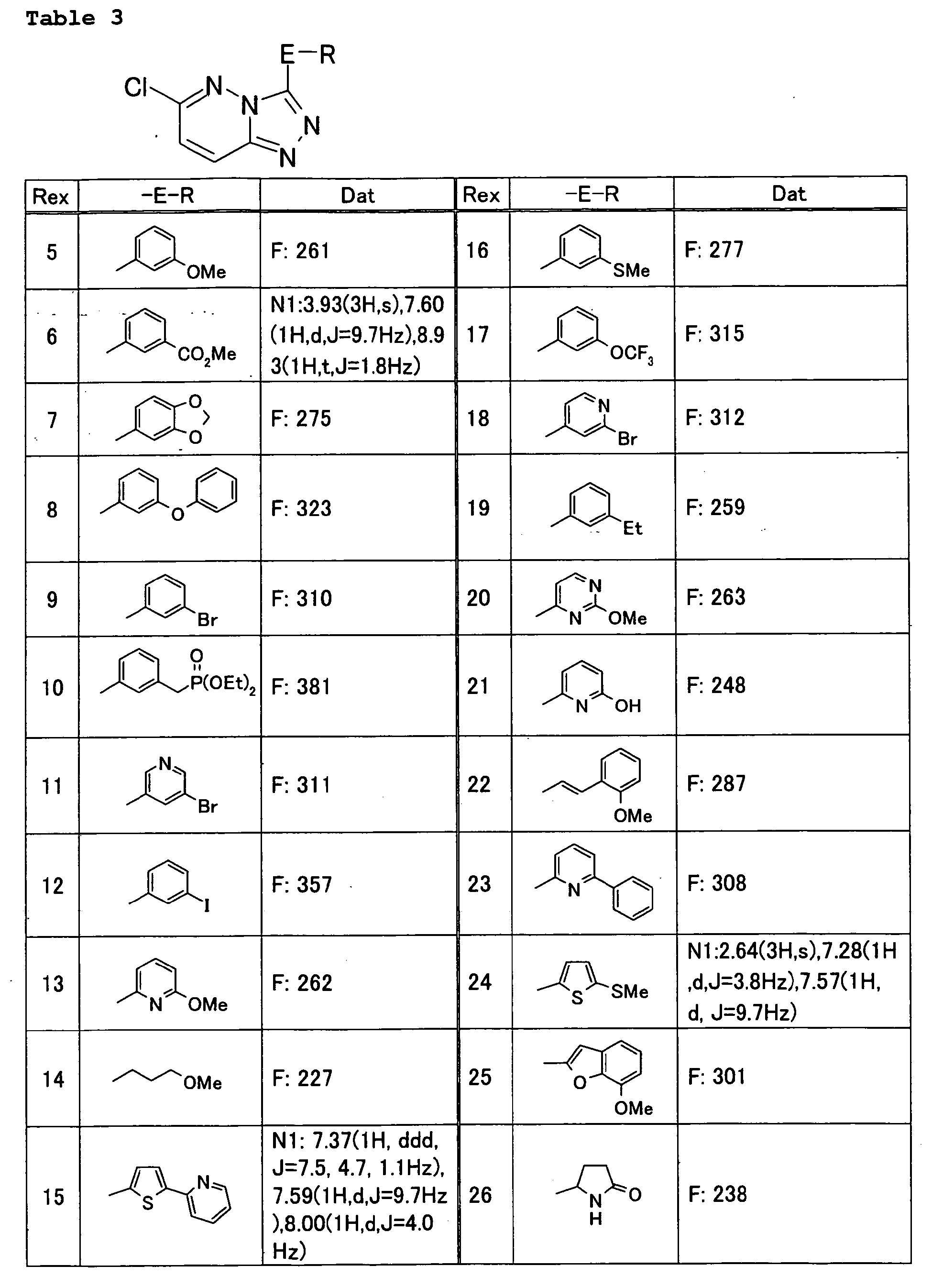

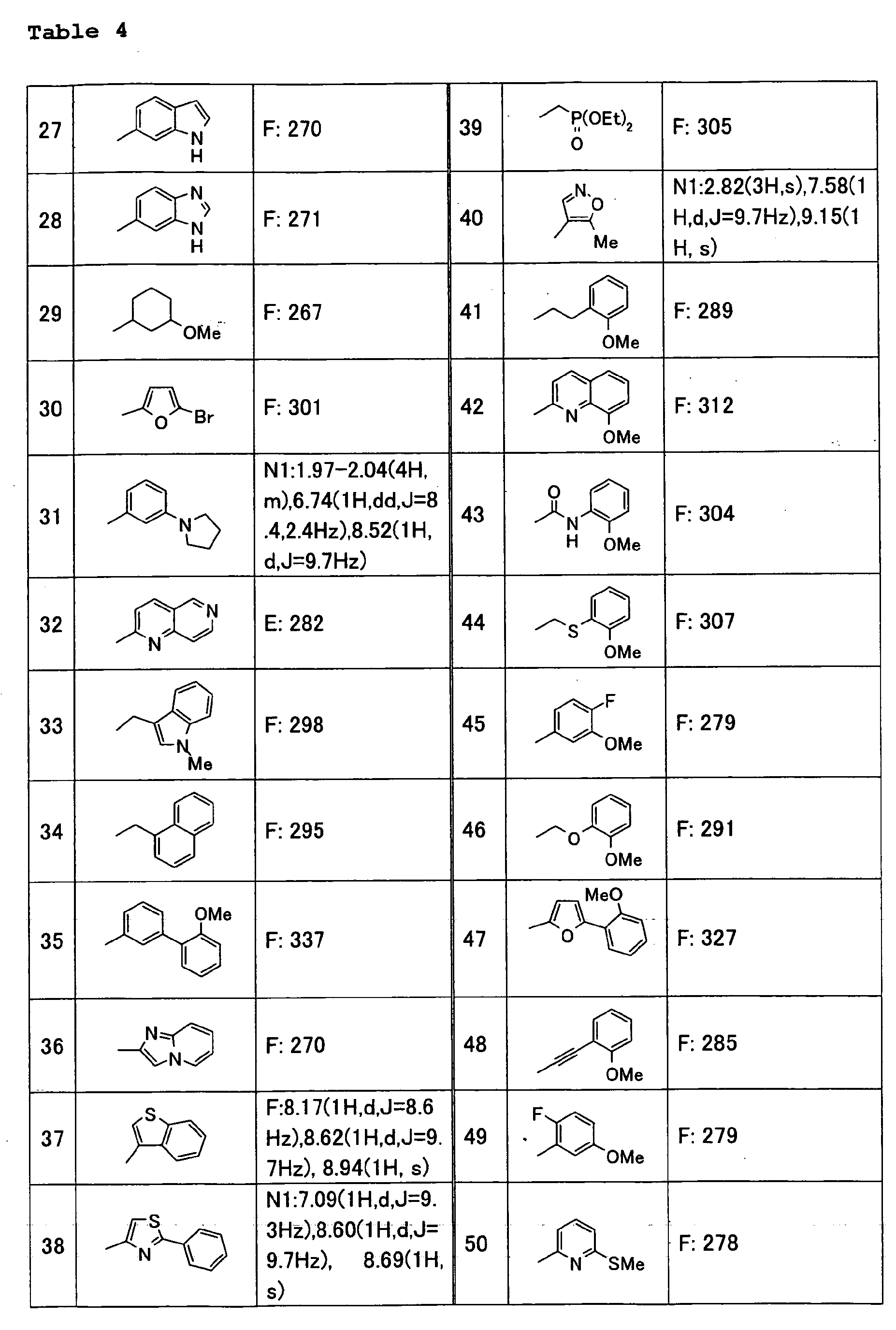

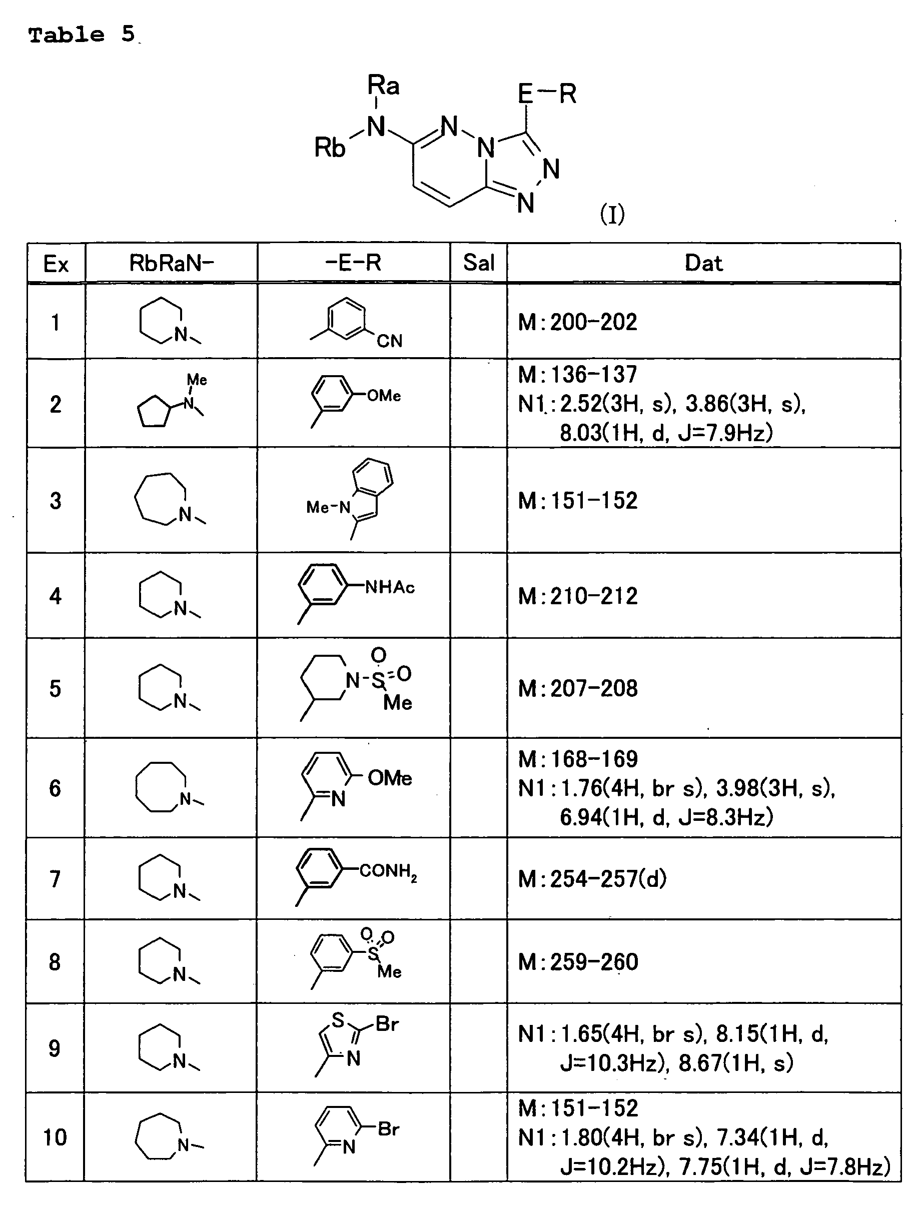

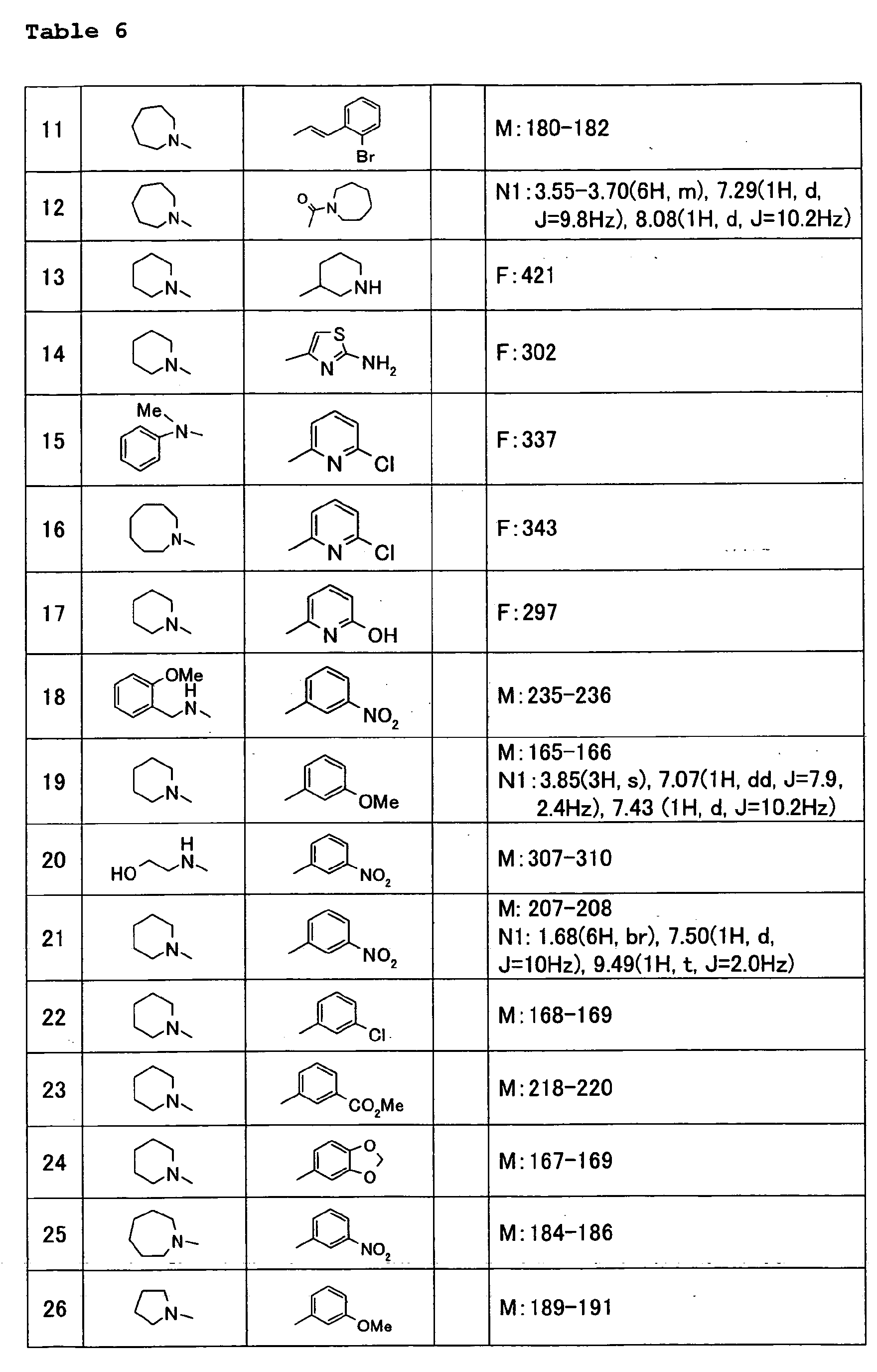

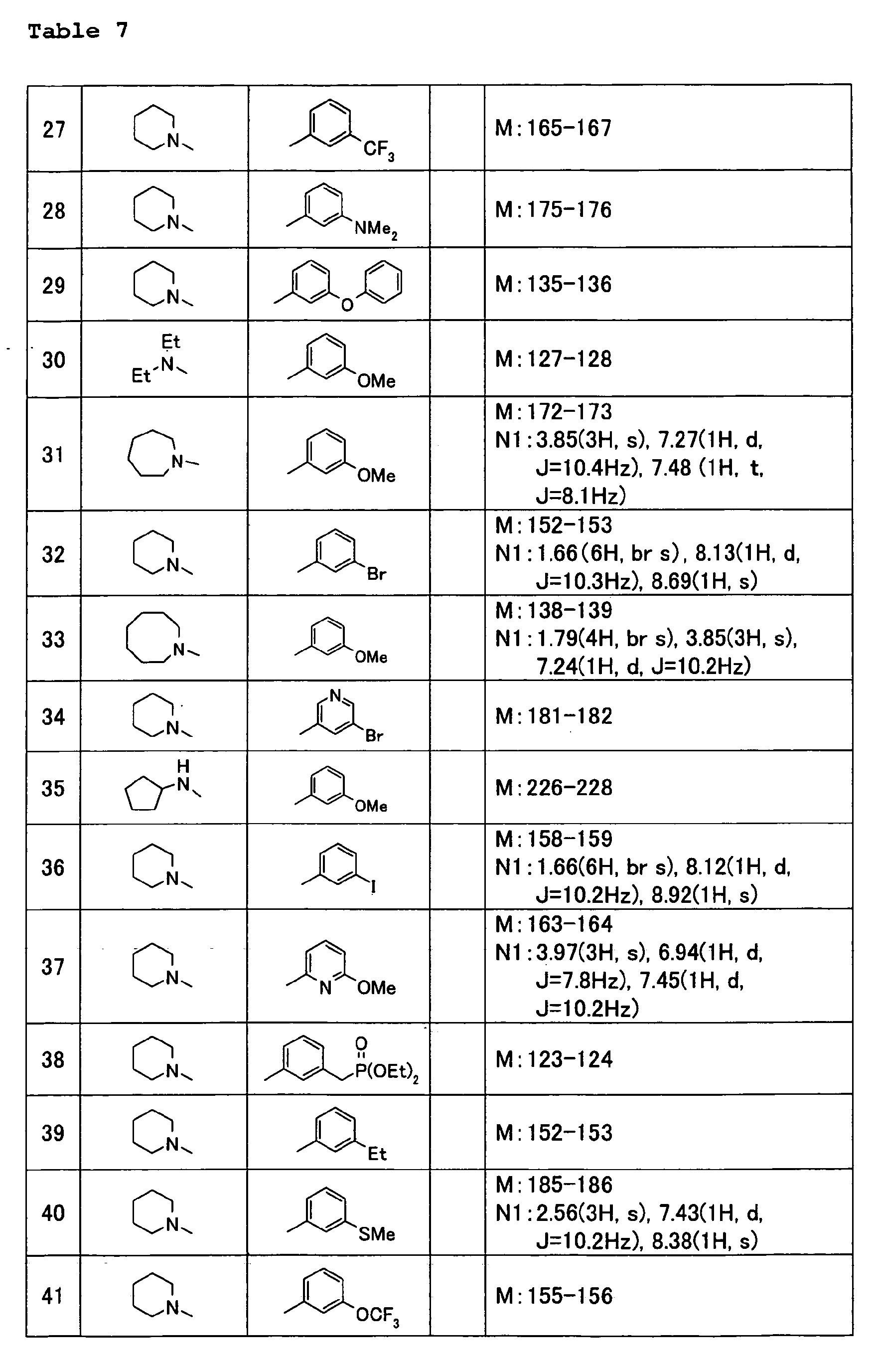

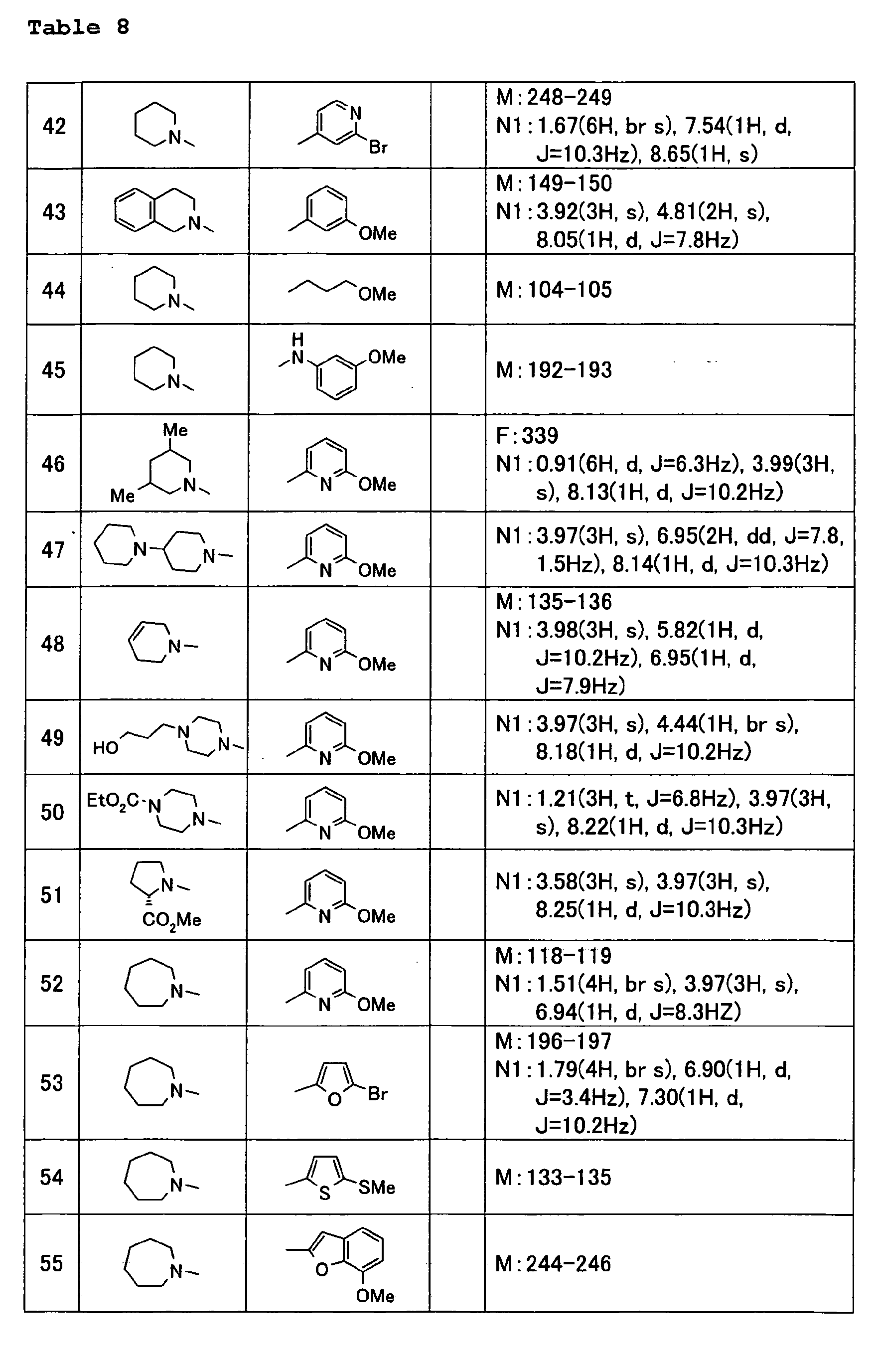

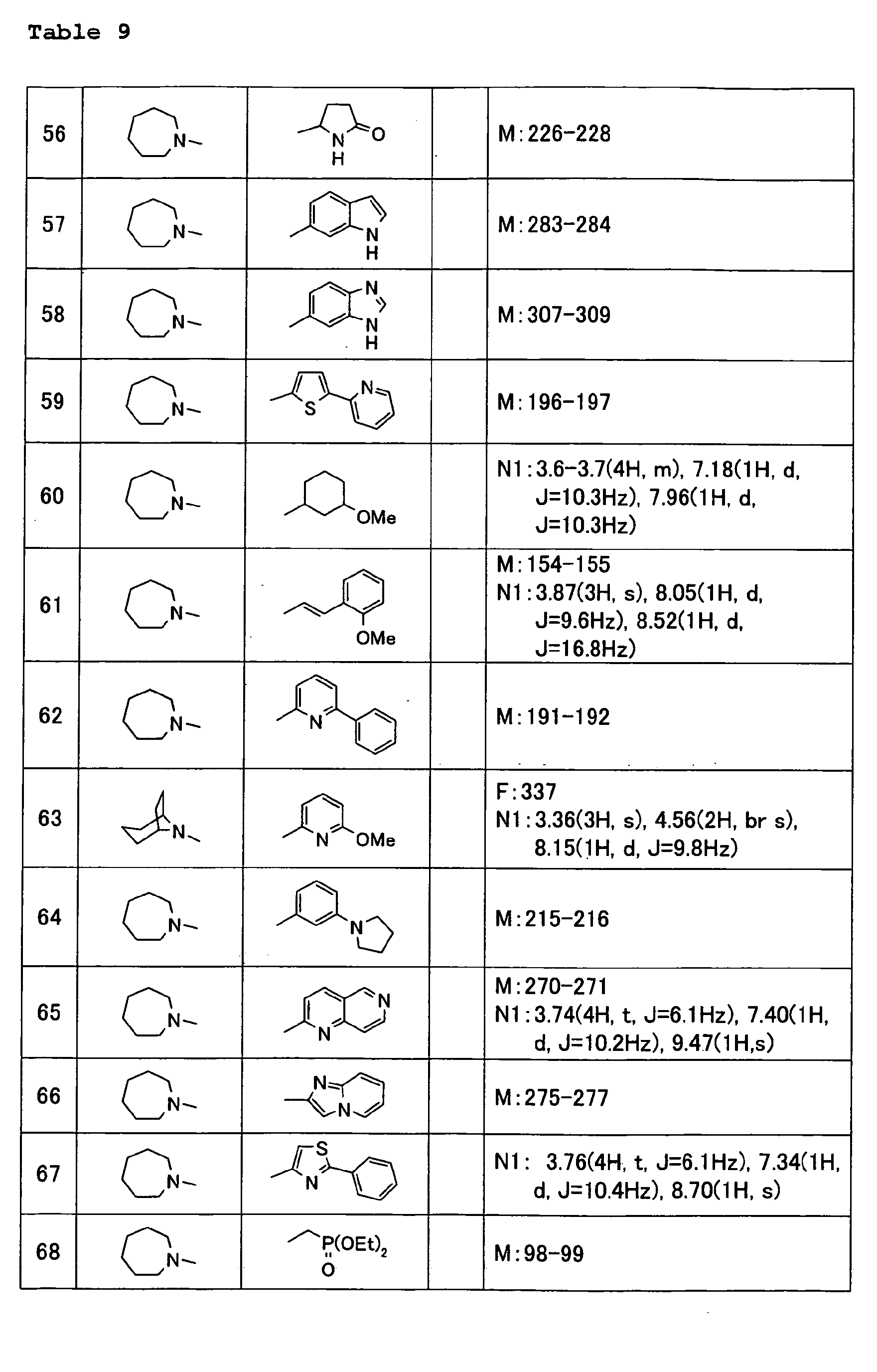

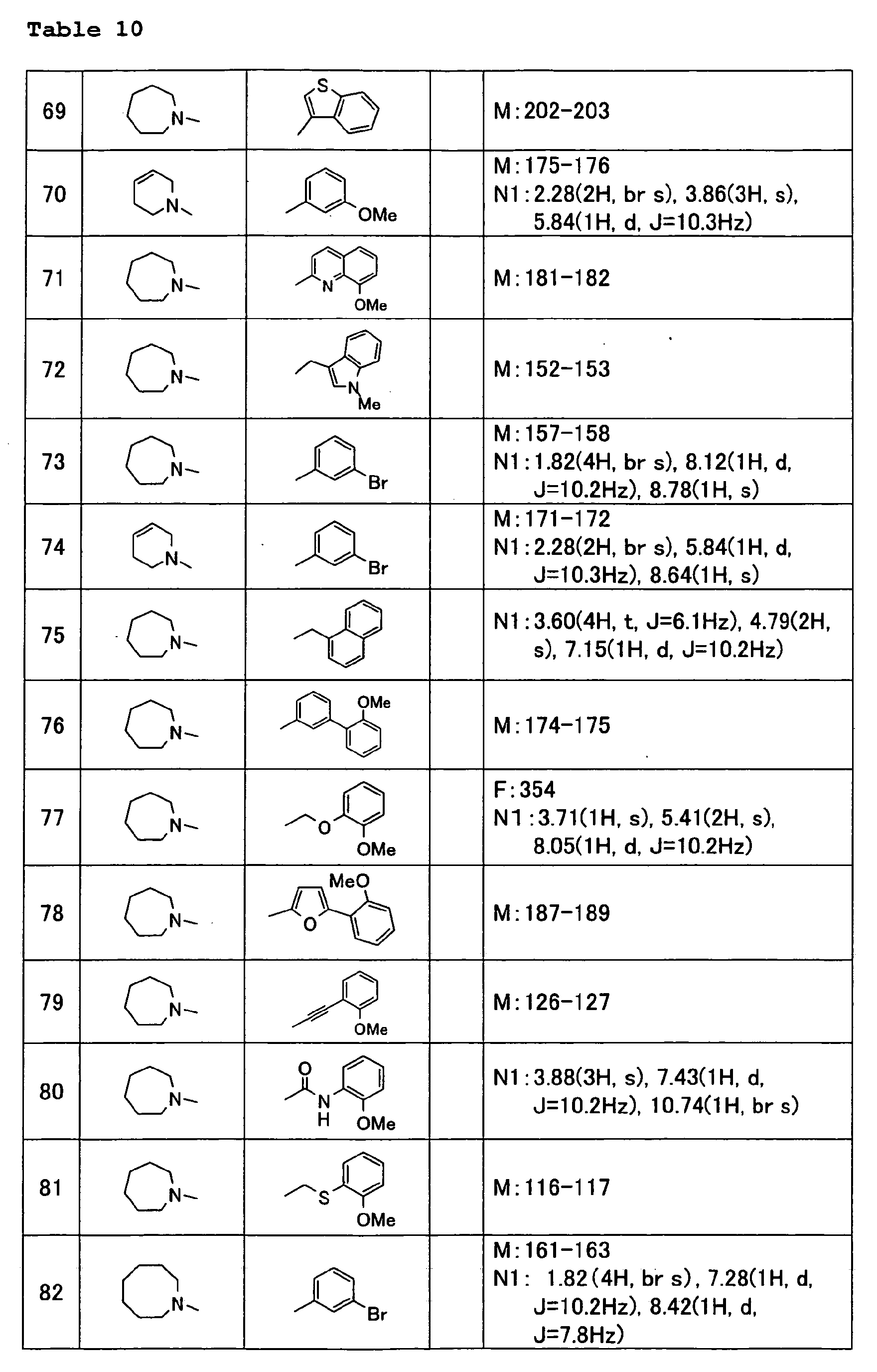

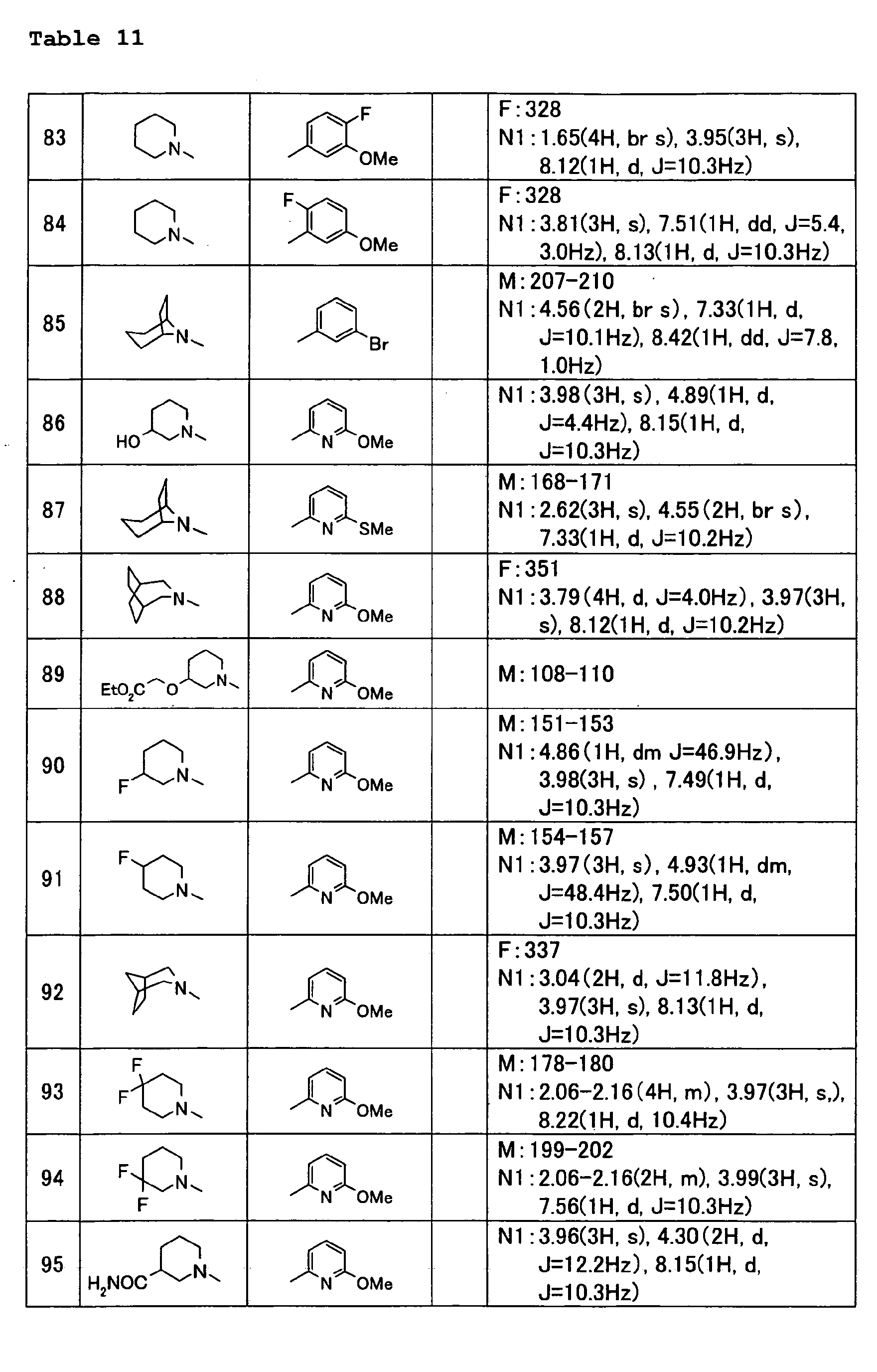

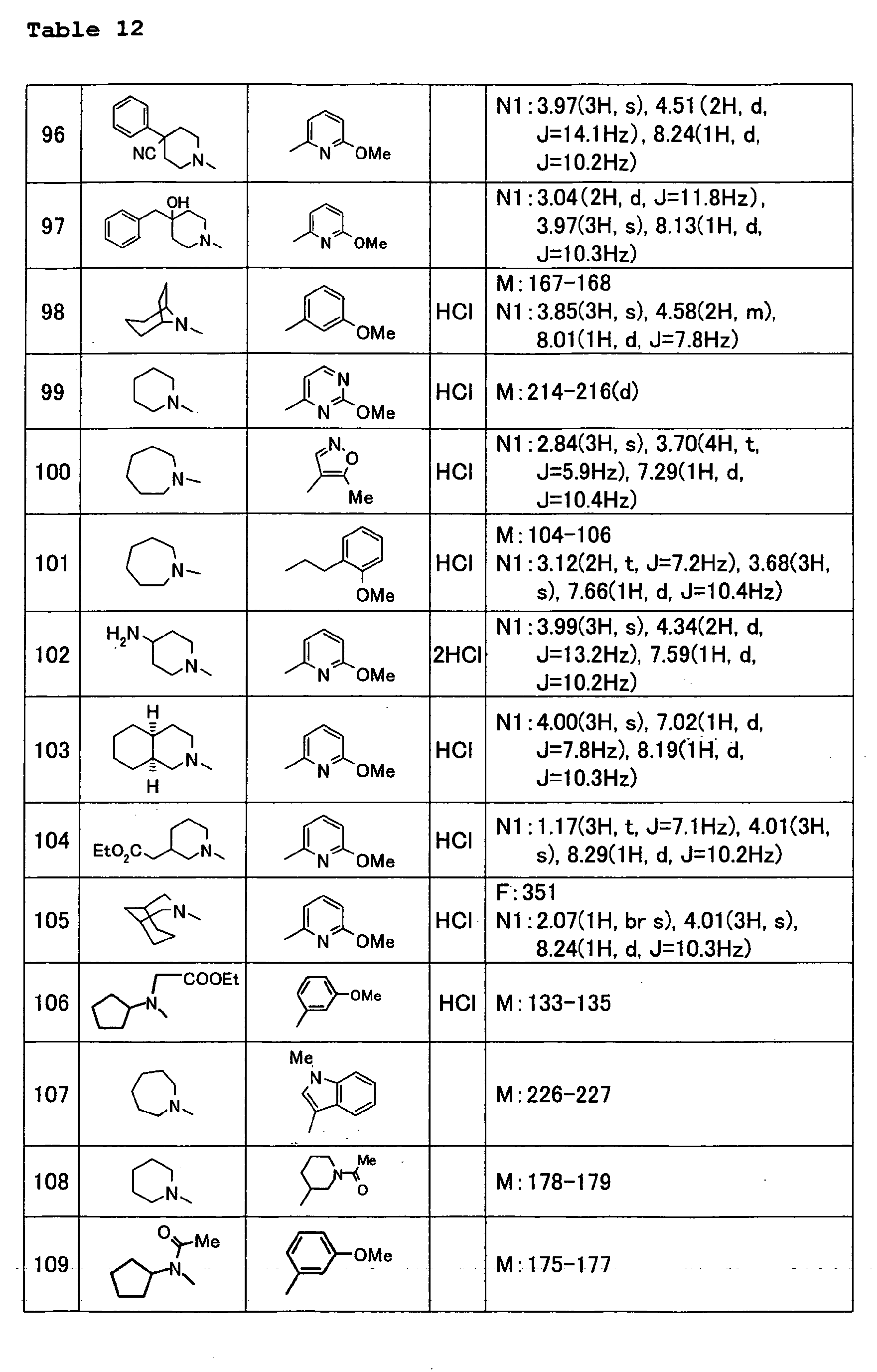

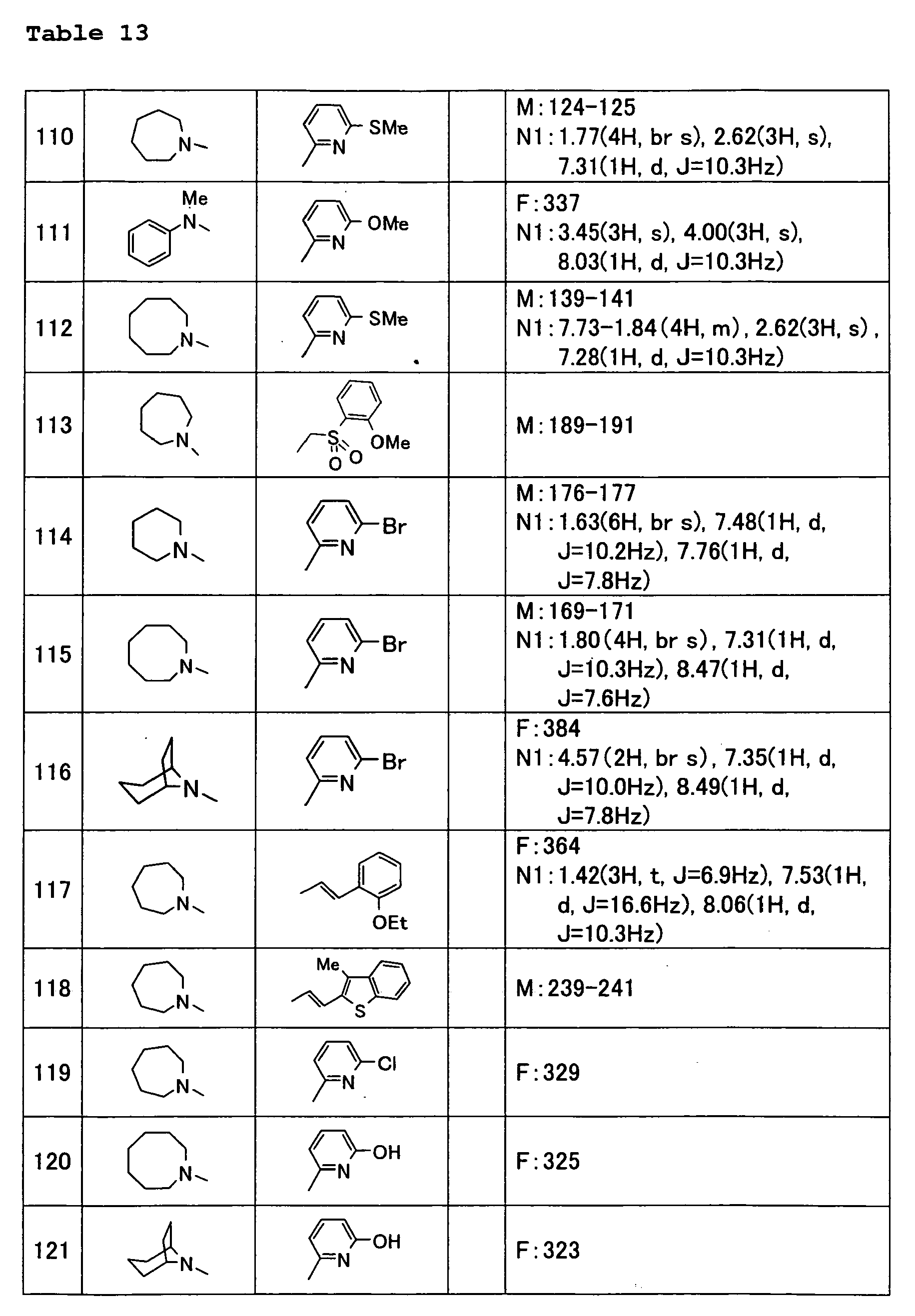

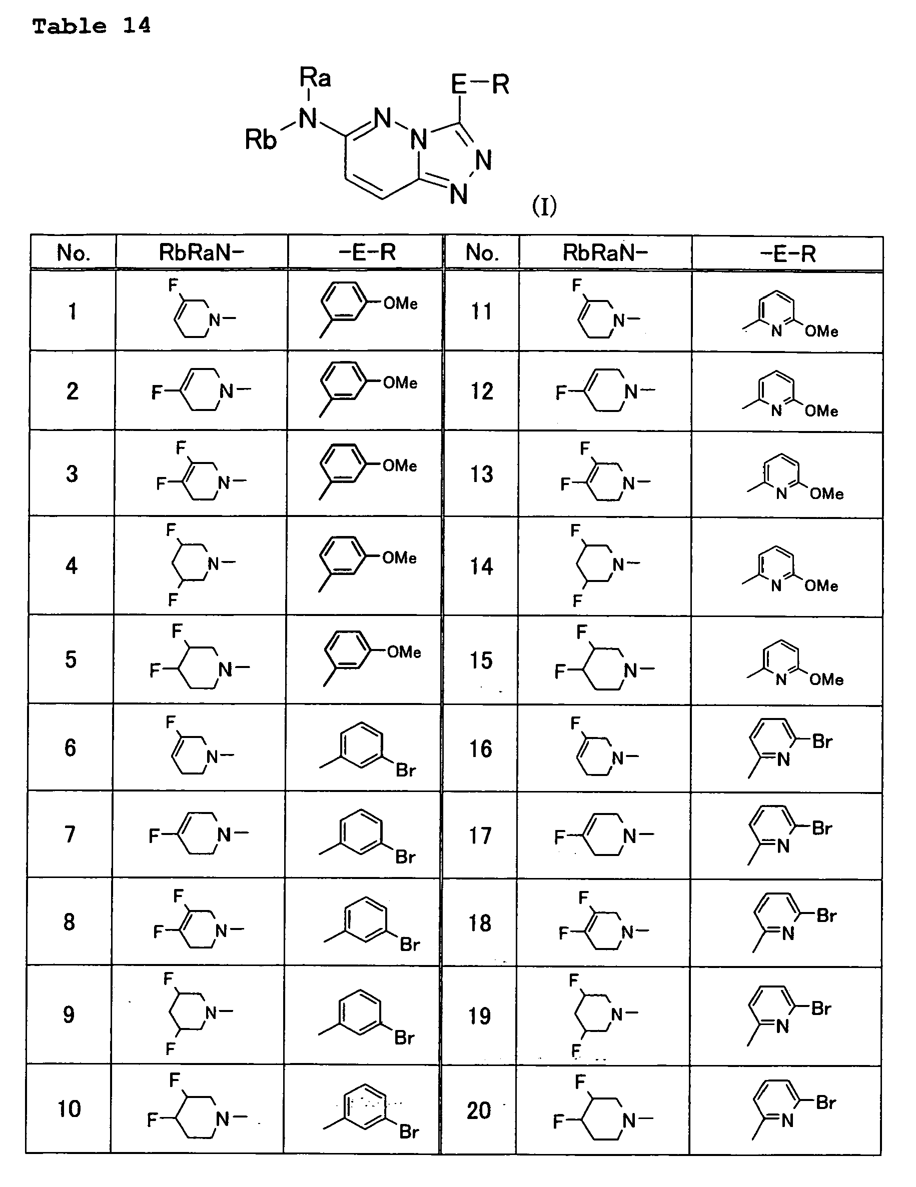

**Example 4: Formulation example**

| | |
|---|---|
| (Capsules) | 1000 capsules |
| Alendronate | 5.0 g |
| Compound A | 30.0 g |
| Lactose | 115.0 g |
| Corn starch | 40.0 g |
| Polyvinyl pyrrolidone K30 | 4.0 g |
| Talc | 5.4 g |
| Magnesium stearate | 0.6 g |
| | 200.0 g |

Alendronate was mixed with 35.0 g of lactose and 5 g of corn starch, and the mixture was kneaded using a paste liquid of polyvinyl pyrrolidone K30 (1.0 g), subjected to granulation by passing through a screen of 20 mesh, dried at 50°C for 2 hours and then passed through a screen of 20 mesh. The compound A was subjected to granulation in the same manner using the remaining lactose, corn starch and paste liquid of polyvinyl pyrrolidone K30. Both of the particles were mixed with talc and magnesium stearate and filled in 200 mg portions into hard capsules to prepare capsules.

### Example 5: Kit

(1) A kit comprising a PTP package containing 1 tablet of an alendronate 35 mg tablet for once a week administration and 7 tablets of a compound A 100 mg tablet for once a day administration
(2) A kit comprising a PTP package containing 1 tablet of an minodronate 1 mg tablet for once a day administration and 2 tablets of a compound A 30 mg tablet for twice a day administration and having display cards on parts close to respective tablet storage spaces, indicating respective administration times

### INDUSTRIAL APPLICABILITY

The pharmaceutical composition or combination product of the invention comprising a non-living body-derived non-peptide osteoblast differentiation promoting compound and a bisphosphonate having a bone resorption inhibitory action is useful as a bone mass increasing inducer which increases the bone mass and/or bone strength by controlling the bone metabolism.

Illustratively, this is useful as preventive or therapeutic agents for osteoporosis, osteitis fibrosa (hyperparathyroidism), osteomalacia, Paget's disease and the like metabolic bone diseases which accompany reduction of the bone mass and/or bone strength; bone tissue restoring agents for bone tissue damages such as bone fracture, re-bone fracture, bone injury, osteoarthropathy, multiple myeloma, bone metastasis and the like; and further, a periodontosis treating agent in the field of dentistry, a restoring agent for periodontal tissue damage, a stabilizer of the artificial root of a tooth, a jaw bank formation promoting agent and the like. Among the preventive or therapeutic agents for osteoporosis and the like metabolic bone diseases, this is expected particularly as a preventive or therapeutic agent for senile osteoporosis having reduced bone formation ability.

Since the preventive effect of the current therapeutic methods which use a bisphosphonate and PTH is said to be insufficient for a clinically serious bone fracture of femoral neck (cortical bone), as shown in the aforementioned Example 2, the pharmaceutical composition or combination product of the invention capable of markedly and particularly increasing the mass and/or strength of cortical bone is clinically markedly useful.

In addition, the agent for reinforcing the bone mass increasing action of bisphosphonate, which uses the non-living body-derived non-peptide osteoblast differentiation promoting compound of the invention as the active ingredient is also useful as an agent for preventing fragile bone microstructure, excess mineral deposition and delay of bone fracture healing (restoring) process, caused by old bone accumulation which may occur under a low bone turnover condition due to long-term administration of bisphosphonate.

## Claims

1. A pharmaceutical composition which is a bone mass increasing inducer, comprising a non-living body-derived non-peptide osteoblast differentiation promoting compound as the first component and a bisphosphonate as the second component.

2. The pharmaceutical composition described in claim 1, wherein the first component is a nitrogen-containing heterocyclic compound represented by the following general formula (I) or a salt thereof 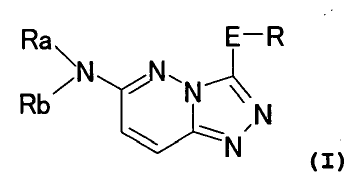 (symbols in the formula have the following meanings,
Ra and Rb: the same or different and each represent H; CO-lower alkyl; SO₂-lower alkyl; an optionally substituted cycloalkyl; an optionally substituted aryl; or a lower alkyl which may have 1 to 3 substituents selected from the group consisting of an optionally substituted cycloalkyl, an optionally substituted aryl, an optionally substituted 4- to 8-membered monocyclic saturated or partially unsaturated heterocyclic ring, CO-lower alkyl, SO₂-lower alkyl, OR¹, SR¹, NR¹R², a halogen, NO₂ CN and COOR¹; provided that at least one of Ra and Rb represents a group other than H; or,
Ra and Rb taken together with an adjacent N atom form a 4- to 8-membered saturated or partially unsaturated heterocyclic ring containing 1 or 2 N atoms as heteroatoms, said heterocyclic ring may be fused with a benzene ring or a cycloalkyl ring and may have a bridge and may form a spiro ring, and said heterocyclic ring may have from 1 to 5 substituent groups,
E: a single bond, a C₁₋₃ alkylene, vinylene (-C=C-), ethynylene (-C≡C-) , CO, NR³, CH₂-J, CONR⁴ or NR⁵CO,
J: O, S, NR⁶, CO, SO or SO₂,
R: an optionally substituted aryl, an optionally substituted heteroaryl, an optionally substituted cycloalkyl, an optionally substituted cycloalkenyl or an optionally substituted 4- to 8-membered monocyclic saturated or partially saturated heterocyclic ring,
R¹ to R⁶: the same or different and each denotes H or a lower alkyl;
with the proviso that the following compounds are excluded:
(1) a compound wherein Ra and Rb taken together with an adjacent N atom form a piperidino, E is a single bond and R is a piperidino, unsubstituted phenyl, *p-*(trifluoromethyl)phenyl, *p*-chlorophenyl or *o*-nitrophenyl,
(2) a compound wherein Ra and Rb taken together with an adjacent N atom form a 4-methyl-1-piperazinyl, E is a single bond, and R is an unsubstituted phenyl, *p-*methylphenyl, *m*-methylphenyl, *p*-methoxyphenyl, *m-*chlorophenyl, *p*-chlorophenyl or *m*-nitrophenyl,
(3) a compound wherein R is an optionally substituted imidazolyl, 5-nitro-2-furyl or 5-nitro-2-thienyl,
(4) a compound wherein Ra is H, Rb is cyclopropyl, E is a single bond and R is a *p*-(trifluoromethyl)phenyl, and
(5) a compound wherein Ra is a methyl, Rb is a 2-hydroxypropyl, E is a single bond and R is a 3-pyridyl).

3. The pharmaceutical composition described in claim 2, wherein the first component is a nitrogen-containing heterocyclic compound selected from 6-Azocan-1-yl-3-(6-methoxypyridin-2-yl)-1,2,4-triazolo[4,3-b]pyridazine, 6-azepan-1-yl-3-(6-bromopyridin-2-yl)-1,2,4-triazolo[4,3-b]pyridazine, 3-(3-methoxyphenyl)-6-(piperidin-1-yl)-1,2,4-triazolo[4,3-b]pyridazine, 3-(3-bromophenyl)-6-(piperidin-1-yl)-1,2,4-triazolo[4,3-b]pyridazine, 6-azepan-1-yl-3-(6-methoxypyridin-2-yl)-1,2,4-triazolo[4,3-b]pyridazine, 6-(4-fluoropiperidin-1-yl)-3-(6-methoxypyridin-2-yl)-1,2,4-triazolo[4,3-b]pyridazine, 6-(3-azabicyclo[3.2.1]octan-3-yl)-3-(6-methoxypyridin-2-yl)-1,2,4-triazolo[4,3-b]pyridazine, 6-(4,4-difluoropiperidin-1-yl)-3-(6-methoxypyridin-2-yl)-1,2,4-triazolo[4,3-b]pyridazine, 6-(3,3-difluoropiperidin-1-yl)-3-(6-methoxypyridin-2-yl)-1,2,4-triazolo[4,3-b]pyridazine, 6-azocan-1-yl-3-(6-bromopyridin-2-yl)-1,2,4-triazolo[4,3-b]pyridazine, and 6-(8-azabicyclo[3.2.1]octan-8-yl)-3-(6-bromopyridin-2-yl)-1,2,4-triazolo[4,3-b]pyridazine, or a salt thereof.

4. The pharmaceutical composition described in claim 2, wherein the second component is a bisphosphonate selected from alendronate, risedronate, pamidronate, incadronate, minodronate, ibandronate and zoledronate.

5. The pharmaceutical composition described in any one of claims 1 to 4, wherein the bone mass increasing inducer is a preventive or therapeutic agent for a metabolic bone disease.

6. The pharmaceutical composition described in any one of claims 1 to 4, wherein the bone mass increasing inducer is a preventive or therapeutic agent for a bone metabolism turnover reducing type (type II) osteoporosis.

7. A combination product which is a bone mass increasing inducer consisting of two pharmaceutical preparations of a pharmaceutical preparation containing a non-living body-derived non-peptide osteoblast differentiation promoting compound as the first pharmaceutical preparation and a bisphosphonate as the second pharmaceutical preparation, wherein said first and second pharmaceutical preparations are administered simultaneously or separately.

8. The combination product described in claim 7, wherein the first pharmaceutical preparation is a pharmaceutical preparation comprising a nitrogen-containing heterocyclic compound represented by the general formula (I) of claim 2, or a salt thereof.

9. The combination product described in claim 7 or 8, which is a kit comprising at least two pharmaceutical preparations of a pharmaceutical preparation containing a non-living body-derived non-peptide osteoblast differentiation promoting compound as the first pharmaceutical preparation and a bisphosphonate as the second pharmaceutical preparation.

10. An agent for reinforcing the bone mass increasing effect of a non-living body-derived non-peptide osteoblast differentiation promoting compound, which comprises a bisphosphonate as the active ingredient.

11. The agent described in claim 10, which is an agent for reinforcing the bone mass increasing effect of a nitrogen-containing heterocyclic compound represented by the general formula (I) of claim 2, or a salt thereof.

12. An agent for reinforcing the bone mass increasing effect of a bisphosphonate, which comprises a non-living body-derived non-peptide osteoblast differentiation promoting compound as the active ingredient.

13. The agent described in claim 12, which is an agent for reinforcing the bone mass increasing effect of a bisphosphonate, wherein it uses a nitrogen-containing heterocyclic compound represented by the general formula (I) of claim 2, or a salt thereof, as the active ingredient.

14. Use of a non-living body-derived non-peptide osteoblast differentiation promoting compound and a bisphosphonate for producing a drug which is a bone mass increasing inducer.

15. Use of a non-living body-derived non-peptide osteoblast differentiation promoting compound for producing a drug which induces increase of bone mass by the concomitant use of a bisphosphonate.

16. Use of a bisphosphonate for producing a drug which reinforces bone mass increasing effect of a non-living body-derived non-peptide osteoblast differentiation promoting compound.

17. A method for preventing or treating a metabolic bone disease which accompanies reduction of the bone mass and/or bone strength of the patient, which comprises administering an effective amount of a non-living body-derived non-peptide osteoblast differentiation promoting compound and an effective amount of a bisphosphonate, simultaneously or separately.

18. The method described in claim 17, wherein the metabolic bone disease which accompanies reduction of the bone mass and/or bone strength of the patient is a bone metabolism turnover reducing type (type II) osteoporosis.

19. A method for inducing bone mass gain of a patient, which comprises administering an effective amount of a non-living body-derived non-peptide osteoblast differentiation promoting compound and an effective amount of a bisphosphonate, simultaneously or separately, to a patient who requires increase of the bone mass and/or bone strength.

20. The method described in any one of claims 17 to 19, wherein the non-living body-derived non-peptide osteoblast differentiation promoting compound is a nitrogen-containing heterocyclic compound represented by the general formula (I) of claim 2, or a salt thereof.
